# EUROPEAN PATENT SPECIFICATION

(11) **EP 3 381 473 B1**
(45) Date of publication and mention of the grant of the patent: **09.12.2026**
(21) Application number: 16840921.7
(22) Date of filing: 26.08.2016
(51) Int. Cl.: A61K 31/00, A61K 47/00, A23K 10/00, A61P 43/00, A61K 9/107, A61K 9/08, A61Q 19/06, A61K 9/00, A61K 8/86, A61K 8/34, A61K 8/11, A61K 47/44, A61K 45/06, A61K 36/82, A61K 31/05, A61K 31/12

(54) **PHARMACEUTICAL INJECTION FORMULATION FOR REDUCING LOCALIZED FAT OR REDUCING BODY WEIGHT AND PHARMACEUTICAL COMPOSITION FOR USE IN A METHOD FOR REDUCING LOCALIZED SUBCUTANEOUS FAT OR REDUCING BODY WEIGHT OF A SUBJECT**
PHARMAZEUTISCHE INJEKTIONSFORMULIERUNG ZUR VERRINGERUNG LOKALISIERTER FETTANSAMMLUNGEN ODER ZUR VERRINGERUNG DES KÖRPERGEWICHTS UND PHARMAZEUTISCHE ZUSAMMENSETZUNG ZUR VERWENDUNG IN EINEM VERFAHREN ZUR VERRINGERUNG VON LOKALISIERTEM SUBKUTANEM FETT ODER ZUR VERRINGERUNG DES KÖRPERGEWICHTS EINES SUBJEKTS
FORMULATION PHARMACEUTIQUE INJECTABLE DESTINÉE À RÉDUIRE LES AMAS GRAISSEUX LOCALISÉS OU À DIMINUER LE POIDS CORPOREL, ET COMPOSITION PHARMACEUTIQUE DESTINÉE À ÊTRE UTILISÉE DANS UNE MÉTHODE VISANT À RÉDUIRE LA GRAISSE SOUS-CUTANÉE LOCALISÉE OU À DIMINUER LE POIDS CORPOREL D'UN SUJET

(30) Priority: 28.08.2015 WO PCT/CN2015/088340; 20.11.2015 US 201562257846 P; 25.02.2016 US 201662299702 P
(43) Date of publication of application: 03.10.2018
(73) Proprietor: Caliway Biopharmaceuticals Co., Ltd., New Taipei City 221 (TW); Caliway (USA) Co., Ltd, Wilmington, Delaware 19805 (US)
(72) Inventor: LING, Yu-Fang, New Taipei City 221 (TW)
(74) Representative: Kuhnen & Wacker Patent- und Rechtsanwaltsbüro PartG mbB
(86) International application number: PCT/IB2016/055101
(87) International publication number: WO 2017/037593

(56) References cited:
- WO-A2-2009/158248
- WO-A2-2010/062824
- WO-A2-2014/138426
- WO-A2-2017/037593
- US-A1- 200110 021 703
- US-A1- 2011 151 103
- US-A1- 2013 245 118
- US-B1- 6 383 482
- US-B1- 9 458 086
- KIM ET AL: "THE CURRENT STATUS OF BLADDER PRESERVATION IN THE TREATMENT OF MUSCLE INVASIVE BLADDER CANCER", JOURNAL OF UROL, LIPPINCOTT WILLIAMS & WILKINS, BALTIMORE, MD, US, vol. 164, no. 3, 1 September 2000 (2000-09-01), pages 627 - 632, XP005553631, ISSN: 0022-5347, DOI: 10.1016/S0022-5347(05)67267-9

## Description

### [FIELD OF THE INVENTION]

The present invention relates to a pharmaceutical composition for reducing localized fat, specifically, relates to a pharmaceutical composition comprising drug-containing micelles and resveratrol encapsulated in the micelles, and the pharmaceutical composition is for localized fat reduction.

### [BACKGROUND OF THE INVENTION]

In recent years, more and more people have changed their views of beauty and raised their standards of individual health and body shape. As a result, people are not only concerned about weight loss, but focus more on reducing localized fat or contouring body shape in order to be healthier and achieve better body shape. Furthermore, common weight loss regimens, whether it is through diet or exercise, cannot reduce fat at a specific location. Currently, if wanting to reduce localized fat at specific locations (such as the waist, abdomen, legs, arms, chin, and face, etc.), the only available technology is liposuction.

At present, liposuction is the most prevalent technology to reduce localized fat. However, the procedure of liposuction causes severe damages to the nerves, blood vessels, and other tissues. Liposuction also comes with risks of infections, severe bleeding, prolonged anesthesia, and unpredictable life-threatening conditions such as fat embolism and allergic reactions to anesthesia. In addition, it is common to experience significant bruising and swelling, severe pain, and post-operational recovery can take as long as 3 to 6 months, and the liposuction site may become uneven. Therefore, statistical analyses revealed that even though many people have considered liposuction to improve the accumulation of localized subcutaneous fat or body curves, less than 40 percent of them actually went through liposuction. It shows that the customers who want to improve body curves or reduce localized fat are deterred from the problems of side effects of liposuction, pain after liposuction, or risks of liposuction.

Although some non-surgical localized fat-reducing pharmaceutical compositions or equipments can partially lower the side effects, they are usually not effective and come with other side effects, such as necrosis of surrounding normal cells, inflammation of surrounding tissues, and sharp pain. Additionally, their administration sites are limited. Therefore, the market is eagerly demanding for an effective localized fat-reducing pharmaceutical composition that has less side effects, a better safety profile, and a shorter recovery period. Examples of pharmaceutical compositions comprising resveratrol and/or curcumin that can inter alia treat subcutaneous fat and/or promote weight loss can be found, for example, in WO 2009/158248 A2, WO 2010/062824 A2, US 2013/245118 A1 and Kim et al: "The current status of bladder preservation in the treatment of muscle invasion bladder cancer", Journal of Urology. Vol. 164, no. 3, 2000, pp. 627-632.

In case of the customers and doctors have significant high demand, the pressing concern is to develop a localized fat-reducing pharmaceutical composition that breaks through the limits of current technologies.

### [Summary of The Invention]

In view of the deficiency of prior art, the present invention provides a pharmaceutical composition for reducing localized fat. The pharmaceutical composition comprises drug-containing micelles made of surfactants, and resveratrol encapsulated in said drug-containing micelles. The pharmaceutical composition for reducing localized fat can reduce fat at the administration site, and has the advantages of high stability, high fat tissue bioavailability, few side effects, and sustained release.

The present invention can promote apoptosis of the adipocytes at the administration site, thereby achieving the goal of localized fat reduction at the administration site. The present invention dramatically solves the problems of prior arts, including adverse reactions and side effects such as necrosis of the surrounding cells and inflammation reactions, and its efficacy on localized fat reduction is significantly superior to other non-surgical pharmaceutical compositions for localized fat reduction. The present invention is suitable for administration at sites requiring subcutaneous fat reduction by direct injection, subcutaneous implantation, implanted infusion, or skin absorption ways, e.g. cream or patch application, without the need for surgery or the help with equipment. Preferably, it is subcutaneously injected into the localized subcutaneous fat layer at the administration site. Preferably, the formulations for injection of the pharmaceutical composition in the present invention include, but are not limited to, powder for injection, or powder for solution for injection. The "localized fat" in the present invention includes but is not limited to the waist, abdomen, legs, arms, chin, and face, etc.

In the present invention, the term "resveratrol" refers to resveratrol extracted from natural plants or commercially available resveratrol. Preferably, the purity of resveratrol is 90% to 100%.

In the present invention, the term "green tea extract" refers to green tea ingredient mixture extracted by any solvent with any extraction method, commercially available green tea extract, any mixture with at least 45% (wt.%) of epigallocatechin gallate (EGCG), or commercially available epigallocatechin gallate (EGCG).

In the present invention, the term "micelle" refers to a microstructure formed by surfactants, wherein each of the surfactants has a hydrophilic end and a hydrophobic (lipophilic) end, and the surfactants are arranged in a way that the hydrophilic ends face outward and the hydrophobic (lipophilic) ends face inward to form the microstructure. Preferably, the microstructure is a spherical structure, a spherical-like structure, or other microstructural structures.

In the present invention, the term "drug-containing micelles" refer to micelles containing resveratrol; that is, the term "drug-containing micelles" refer to micelles encapsulating or containing resveratrol.

In the present invention, the term "second lipophilic drug-containing micelles" refer to micelles containing any lipophilic drugs expect resveratrol. That is, the term "second lipophilic drug-containing micelles" refer to micelles encapsulating or containing other lipophilic drugs.

Wherein, the term "other lipophilic drugs" refer to at least one of curcumin, quercetin, puerarin, and other lipophilic drugs except resveratrol, or combination thereof; or, the term "other lipophilic drugs" refer to lipophilic drugs other than resveratrol.

In the present invention, the term "hydrophilic drugs" refer to at least one of green tea extract, epigallocatechin gallate, epicatechin, epicatechin gallate, epigallocatechin, gallocatechin gallate, gallocatechin, catechin gallate, catechin, epigallocatechin gallate (EGCG), caffeine, carnitine, L-carnitine, synephrine, chlorogenic acid, and other hydrophilic drugs, or combination thereof.

In the present invention, the term "state without precipitation", as used herein, refers to a state wherein no precipitation can be observed with the naked eye, that is, without the need by the assistance of artificial instruments.

The present invention provides an injection formulation as disclosed in claim 1 for reducing localized fat or reducing body weight, comprising drug-containing micelles; resveratrol encapsulated in said drug-containing micelles; wherein the drug-containing micelle is a microstructure formed by a non-ionic surfactant selected from the group consisting of a pharmaceutically acceptable polyoxyethylene castor oil, a polyoxyl 15 hydroxystearate (HS-15), and a combination thereof. The hydrophilic-lipophilic balance value (HLB value) of the non-ionic surfactant is greater than 10. The pharmaceutically acceptable polyoxyethylene castor oil comprises polyoxyl 35 castor oil (ELP). When the non-ionic surfactant comprises the ELP, the weight ratio of the resveratrol to the ELP in the formulation is 1:5 to 1:500. When the non-ionic surfactant comprises HS-15, the weight ratio of the resveratrol to the HS-15 in the formulation is 1:40. The drug-containing micelles do not comprise unsaturated fatty acid.

In a preferred embodiment, the diameter of the drug-containing micelles is 3~250 nm.

In a preferred embodiment, the diameter of the drug-containing micelles is 5~50 nm.

In a preferred embodiment, the concentration of the resveratrol in the injection formulation is 0.2~ 166.7 mg/mL, optionally the concentration of resveratrol in the injection formulation is 2.5~ 60 mg/mL.

In a preferred embodiment, the injection formulation further comprises a pharmaceutically acceptable aqueous solution. In a preferred embodiment, the injection formulation further comprises a second lipophilic drug-containing micelle; wherein the second lipophilic drug-containing micelle is another microstructure formed by a second non-ionic surfactant, and a second lipophilic drug, which is encapsulated in said second lipophilic drug-containing micelle. Optionally, a) the second non-ionic surfactant is selected from the group consisting of polysorbate 80, a polyoxyethylene castor oil, polyoxyl 15 hydroxystearate (HS 15), and other non-ionic surfactant, or a combination thereof; further optionally wherein the polyoxyethylene castor oil is at least one of polyoxyl 35 castor oil (ELP), polyoxyl 40 hydrogenated castor oil (RH 40), and other polyoxyethylene castor oils, or combination thereof, or b) the second lipophilic drug is at least one of quercetin, synephrine, puerarin, curcuminoid, and other lipophilic drug except resveratrol, or combination thereof, further optionally wherein the weight ratio of resveratrol to the second lipophilic drugs is 30:1 to 1:20.

In a preferred embodiment, the injection formulation further comprises a hydrophilic drug, optionally the hydrophilic drug is at least one of green tea extract, epigallocatechin gallate, epicatechin, epicatechin gallate, epigallocatechin, gallocatechin gallate, gallocatechin, catechin gallate, catechin, epigallocatechin gallate (EGCG), caffeine, carnitine, L-carnitine, synephrine, chlorogenic acid, and other hydrophilic drug, or combination thereof, further optionally
a) the weight ratio of resveratrol to the hydrophilic drugs is 20:1 to 1:30,
b) wherein the hydrophilic drug is epigallocatechin gallate, and the concentration of epigallocatechin gallate in the injection formulation is 0.25~ 300 mg/mL, or
c) the hydrophilic drug is green tea extract, and the weight ratio of resveratrol to the green tea extract is 30:1 to 1:30.
The present invention further provides a pharmaceutical composition for use in a method for reducing localized subcutaneous fat or reducing body weight of a subject, wherein the pharmaceutical composition is set forth above, and wherein the method comprising a step of injecting the pharmaceutical composition to a local site of the subject.

In a preferred embodiment, the pharmaceutical composition further comprises the hydrophilic drug,
a) wherein the hydrophilic drug is epigallocatechin gallate, and the concentration of epigallocatechin gallate in the pharmaceutical composition is 0.25~ 300 mg/mL, or
b) the hydrophilic drug is green tea extract, and the weight ratio of resveratrol to the green tea extract is 30:1 to 1:30; preferably wherein the amount of the surfactant is 0.24 ~ 70 parts by weight, based on 1 part by weight of the total amount of resveratrol and green tea extract; or the weight ratio of the total weight of the resveratrol and the green tea extract to the surfactant is 4:1 to 1:70.

In a preferred embodiment,
a) the pharmaceutical composition is to be injected at the local site at a dose of 0.2 ~ 20 mg/cm²,
b) the pharmaceutical composition is to be administrated at the local site at a dose of 0.2 ~ 40 mg/kg, or
c) the dosing frequency of the pharmaceutical composition is 1 to 12 times every other day to every 30 days.

In a preferred embodiment, the subject is an animal or a human.

### [Brief Description of the Drawings]

Figure 1A: A bar graph showing the effects of the non-micellar resveratrol solution for subcutaneous injection and the non-micellar green tea extract solution for subcutaneous injection on the amount of subcutaneous fat of rats
Figure 1B: A bar graph showing the effects of the non-micellar resveratrol solution for subcutaneous injection and the non-micellar green tea extract solution for subcutaneous injection on the relative weight gain of rats.
Figure 2A : A bar graph showing the effects of resveratrol subcutaneous injection formulations prepared with different excipients on the amount of localized subcutaneous fat of rats.
Figure 2B : A bar graph showing the effects of resveratrol subcutaneous injection formulations prepared with different excipients on the relative weight gain of rats.
Figure 3: A bar graph showing the effects of the resveratrol pharmaceutical composition on the amount of subcutaneous fat of rats.
Figure 4A: A bar graph showing the effects of the resveratrol complex pharmaceutical composition on the amount of subcutaneous fat of rats.
Figure 4B: A bar graph showing the effects of the resveratrol complex pharmaceutical composition on the relative weight gain of rats.
Figure 5: The effects of the resveratrol-other lipophilic drug complex pharmaceutical composition on the apoptosis of the mature adipocytes.
Figure 6: The effects of the resveratrol-other hydrophilic drug complex pharmaceutical composition on the apoptosis of the mature adipocytes.

### [Detailed Description of The Invention]

Resveratrol is a polyphenol commonly found in the skin of red grapes, Japanese knotweed, or red wine. Because resveratrol has low solubility in aqueous solutions, it is easily to be rapidly metabolized into glucuronide metabolites and sulfate metabolites in the body, it is rapidly excreted via urine and feces, and it is extremely poor bioavailability, development of resveratrol-based pharmaceutical compositions faces a certain degree of difficulties. The inventor also faced these obstacles at the early stage of developing this pharmaceutical composition.

A pharmaceutical composition for reducing localized fat, comprising: drug-containing micelles, wherein the drug-containing micelles are evenly distributed in said pharmaceutically acceptable aqueous solution; and resveratrol encapsulated in said drug-containing micelles.

A pharmaceutical composition may further comprise a pharmaceutically acceptable aqueous solution, and the drug-containing micelles are evenly distributed in said pharmaceutically acceptable aqueous solution. The pharmaceutically acceptable aqueous solution is water for injection, an aqueous solution for injection, or normal saline.

A surfactant is a non-ionic surfactant.

A non-ionic surfactant may be at least one of polysorbate 80 (tween 80), solutol HS 15 (polyoxyl 15 hydroxystearate, 2-hydroxyethyl 12-hydroxyoctadecanoate), polyoxyethylene castor oils, and other non-ionic surfactants, or combination thereof.

Polyoxyethylene castor oils are at least one of Kolliphor ELP (also known as polyoxyl 35 castor oil, polyoxyl 35 hydrogenated castor oil, or cremophor ELP), cremophor RH 40, and other polyoxyethylene castor oils, or combination thereof.

A weight ratio of resveratrol to the surfactant is 1:4 to 1:500.

A weight ratio of resveratrol to the surfactant is 1:5 to 1:200.

A weight ratio of resveratrol to the surfactant is 1:8 to 1:80.

A pharmaceutical composition maintains at a state without precipitation for at least 24 hours when it is subjected to accelerated stability test at 25°C ±2°C, relative humidity 60% ± 5%, and in the absence of direct light.

A pharmaceutical composition maintains at a state without precipitation for at least 6 months when it is subjected to accelerated stability test at 25°C ±2°C, relative humidity 60% ± 5%, and in the absence of direct light.

A concentration of resveratrol in the pharmaceutical composition is 0.2 ~ 166.7 mg/mL.

A concentration of resveratrol in the pharmaceutical composition is 2.5 ~ 60 mg/mL.

A pharmaceutical composition further comprises green tea extract, and the green tea extract is dissolved in the pharmaceutically acceptable aqueous solution; wherein the green tea extract comprises:
a first green tea extract ingredient; wherein said first green tea extract ingredient is epigallocatechin gallate (EGCG).

A concentration of epigallocatechin gallate (EGCG) in the pharmaceutical composition is 0.25 ~ 300 mg/mL.

A concentration of epigallocatechin gallate (EGCG) in the pharmaceutical composition is 1~200 mg/ mL.

A content of epigallocatechin gallate (EGCG) is 45 ~ 100% by weight (wt.%), based on 100% by weight of the total green tea extract.

A weight ratio of the resveratrol to the green tea extract is 30:1 to 1:30.

A weight ratio of the resveratrol to the green tea extract is 20:1 to 1:20.

A weight ratio of the resveratrol to the green tea extract is 10:1 to 1:10.

An amount of the surfactant is 0.24 ~ 70 parts by weight, based on 1 part by weight of the total amount of resveratrol and green tea extract; or, the weight ratio of the total weight of the resveratrol and the green tea extract to the surfactant is 4:1 to 1:70.

A pharmaceutical composition further comprises a pharmaceutically acceptable aqueous solution and a second lipophilic drug-containing micelle, wherein the second lipophilic drug-containing micelles are evenly distributed in the pharmaceutically acceptable aqueous solution.

A second lipophilic drug-containing micelle is another microstructure formed by a second surfactant, and an other lipophilic drug (or second lipophilic drug) is encapsulated in said second lipophilic drug-containing micelle.

A hydrophilic-lipophilic balance value (HLB value) of the second surfactant is greater than 10.

A second surfactant is a non-ionic surfactant.

A non-ionic surfactant is at least one of polysorbate 80 (tween 80), solutol HS 15 (polyoxyl 15 hydroxystearate, 2-hydroxyethyl 12-hydroxyoctadecanoate), polyoxyethylene castor oils, and other non-ionic surfactants, or combination thereof.

A polyoxyethylene castor oil is at least one of cremophor ELP, cremophor RH 40, and other polyoxyethylene castor oils, or combination thereof.

A lipophilic drug is at least one of quercetin, synephrine, puerarin, curcumin, and other lipophilic drugs except resveratrol, or combination thereof.

A weight ratio of the resveratrol to the second lipophilic drug is 30:1~1:20.

A weight ratio of the resveratrol to the second lipophilic drug is 20:1~1:15.

A weight ratio of the resveratrol to the second lipophilic drug is 15:1~1:10.

A pharmaceutical composition further comprises a pharmaceutically acceptable aqueous solution and a hydrophilic drug.

A hydrophilic drug is at least one of green tea extract, epigallocatechin gallate, epicatechin, epicatechin gallate, epigallocatechin, gallocatechin gallate, gallocatechin, catechin gallate, catechin, epigallocatechin gallate (EGCG), caffeine, carnitine, L-carnitine, synephrine, chlorogenic acid, and other hydrophilic drugs, or combination thereof.

A weight ratio of the resveratrol to the hydrophilic drugs is 20:1 ~ 1:30.

A weight ratio of the resveratrol to the hydrophilic drugs is 15:1 ~ 1:20.

A weight ratio of the resveratrol to the hydrophilic drugs is 10:1 ~ 1:15.

A hydrophilic drug is gallocatechin gallate, and the concentration of gallocatechin gallate in the pharmaceutical composition is 0.25~ 300 mg/mL.

A hydrophilic drug is gallocatechin gallate, and the concentration of gallocatechin gallate in the pharmaceutical composition is 1~ 200 mg/mL.

A hydrophilic drug is green tea extract, and the weight ratio of the resveratrol to the green tea extract is 30:1 to 1:30.

A hydrophilic drug is green tea extract, and the weight ratio of the resveratrol to the green tea extract is 20:1 to 1:20.

A hydrophilic drug is green tea extract, and the weight ratio of the resveratrol to the green tea extract is 10:1 to 1:10.

A pharmaceutical composition further comprises a cosolvent to enhance the solubility of the drug.

A cosolvent is at least one of polyethylene glycol, propylene glycol, ethanol, and other cosolvent, or combination thereof.

A polyethylene glycol is at least one of PEG 200, PEG 400, PEG 600, and other polyethylene glycol, or combination thereof.

A pharmaceutical composition further comprises a suspending agent to decrease the sedimentation rate of the drug or micelles.

A suspending agent is at least one of sodium alginate, glycerol, carboxymethylcellulose sodium, mannitol, and other suspending agent, or combination thereof.

A pharmaceutical composition further comprises an oil phase excipient to enhance the stability of the pharmaceutical composition and the solubility of the drug.

An oil phase excipient is at least one of unsaturated fatty acid, glycerol, triglyceride, and other oil phase excipient, or combination thereof.

An unsaturated fatty acid is at least one of oleic acid, castor oil, sesame oil, cottonseed oil, soybean oil, safflower oil, corn oil, and other unsaturated fatty acid, or combination thereof.

A triglyceride is at least one of medium chain triglycerides and other triglyceride, or combination thereof.

A pharmaceutically acceptable aqueous solution comprises a local anesthetic.

A local anesthetic is at least one of amides, para-aminobenzoic acid esters, and amino ethers, or combination thereof.

Amides are at least one of dibucaine, lidocaine, mepivacaine HCl, bupivacine HCl, pyrrocaine HCl, prilocaine HCl, digammacaine, and oxethazaine, or combination thereof.

Para-aminobenzoic acid esters are at least one of butacaine, dimethocaine, and tutocaine, or combinations thereof.

Amino ethers are at least one of quinisocaine and pramocaine, or combination thereof.

A pharmaceutically acceptable aqueous solution comprises an antioxidant.

An antioxidant is at least one of beta-carotene, lutein, lycopene, bilirubin, vitamin A, vitamin C (ascorbic acid), vitamin E, uric acid, nitric oxide, nitroxide, pyruvate, catalase, superoxide dismutase, glutathione peroxidases, N-acetyl cysteine, and naringenin, or combination thereof.

A formulation for subcutaneous fat layer injection or a formulation for subcutaneous injection for reducing the amount of localized subcutaneous fat, comprising:
a pharmaceutically acceptable aqueous solution,
drug-containing micelles, wherein the drug-containing micelles are evenly distributed in the pharmaceutically acceptable aqueous solution, and
resveratrol encapsulated in said drug-containing micelles;
wherein said drug-containing micelle is a microstructure formed by a pharmaceutically acceptable surfactant, and the hydrophilic-lipophilic balance value (HLB value) of the pharmaceutically acceptable surfactant is greater than 10.

An amount of localized subcutaneous fat is the amount of subcutaneous fat at the administration site.

A diameter of the drug-containing micelles is 3 ~250 nm.

A diameter of the drug-containing micelles is 5~50 nm.

A pharmaceutically acceptable aqueous solution is water for injection, aqueous solutions for injection, or normal saline.

A surfactant is a non-ionic surfactant.

A non-ionic surfactant is at least one of polysorbate 80 (tween 80), solutol HS 15 (polyoxyl 15 hydroxystearate, 2-hydroxyethyl 12-hydroxyoctadecanoate), polyoxyethylene castor oils, and other non-ionic surfactants, or combination thereof.

Polyoxyethylene castor oils are at least one of cremophor ELP, cremophor RH 40, and other polyoxyethylene castor oils, or combination thereof.

A weight ratio of resveratrol to the surfactant is 1:4 to 1:500.

A weight ratio of resveratrol to the surfactant is 1:5 to 1:200.

A weight ratio of resveratrol to the surfactant is 1:8 to 1:80.

A concentration of resveratrol in the formulation for subcutaneous fat layer injection or the formulation for subcutaneous injection is 0.2 ~ 166.7 mg/mL.

A concentration of resveratrol in the formulation for subcutaneous fat layer injection or the formulation for subcutaneous injection is 2.5 ~ 60 mg/mL.

A formulation for subcutaneous fat layer injection or the formulation for subcutaneous injection further comprises green tea extract, and the green tea extract is dissolved in the pharmaceutically acceptable aqueous solution; wherein the green tea extract comprises:
a first green tea extract ingredient, wherein said first green tea extract ingredient is epigallocatechin gallate (EGCG).

A concentration of epigallocatechin gallate (EGCG) in the formulation for subcutaneous fat layer injection or the formulation for subcutaneous injection is 0.25 ~ 300 mg/mL.

A concentration of epigallocatechin gallate (EGCG) in the formulation for subcutaneous fat layer injection or the formulation for subcutaneous injection is 1 ~ 200 mg/mL.

A content of epigallocatechin gallate (EGCG) is 45 ~ 100% by weight (wt.%) based on 100% by weight of the total green tea extract.

A weight ratio of the resveratrol to the green tea extract is 30:1 to 1:30.

A weight ratio of the resveratrol to the green tea extract is 20:1 to 1:20.

A weight ratio of the resveratrol to the green tea extract is 10:1 to 1:10.

An amount of the surfactant is 0.24 ~ 70 parts by weight, based on 1 part by weight of the total amount of resveratrol and green tea extract; or, the weight ratio of the total weight of the resveratrol and the green tea extract to the surfactant is 4:1 to 1:70.

An administered dosage be injected at the administration site is 0.2 ~ 20 mg/cm².

An administered dosage be injected at the administration site is 0.4 ~ 12 mg/cm².

An administered dosage is 0.2 ~ 40 mg/kg at the administration site.

An administered dosage is 0.4 ~ 20 mg/kg at the administration site.

A dosing frequency is 1 to 12 times every other day to every 30 days.

A dosing frequency is 1 to 8 times every other day to every 21 days.

A formulation for subcutaneous fat layer injection or a formulation for subcutaneous injection further comprises a second lipophilic drug-containing micelle, and the second lipophilic drug-containing micelles are evenly distributed in the pharmaceutically acceptable aqueous solution.

A second lipophilic drug-containing micelle is a microstructure formed by another surfactant, and the other lipophilic drug is encapsulated in said second lipophilic drug-containing micelle.

A hydrophilic-lipophilic balance value (HLB value) of the other surfactant is greater than 10.

Another surfactant is a non-ionic surfactant.

A non-ionic surfactant is at least one of polysorbate 80 (tween 80), solutol HS 15 (polyoxyl 15 hydroxystearate, 2-hydroxyethyl 12-hydroxyoctadecanoate), polyoxyethylene castor oil, and other non-ionic surfactants, or combination thereof.

Polyoxyethylene castor oils are at least one of cremophor ELP, cremophor RH 40, and other polyoxyethylene castor oils, or combination thereof.

A lipophilic drug is at least one of curcumin, quercetin, puerarin, and other lipophilic drugs except resveratrol, or combination thereof.

A pharmaceutically acceptable aqueous solution further comprises a hydrophilic drug.

A hydrophilic drug is at least one of green tea extract, epigallocatechin gallate, epicatechin, epicatechin gallate, epigallocatechin, gallocatechin gallate, gallocatechin, catechin gallate, catechin, epigallocatechin gallate (EGCG), caffeine, carnitine, L-carnitine, synephrine, chlorogenic acid, and other hydrophilic drugs, or combination thereof.

A pharmaceutically acceptable aqueous solution comprises a local anesthetic.

A local anesthetic is at least one of amides, para-aminobenzoic acid esters, amino ethers, or combination thereof.

Amides are at least one of dibucaine, lidocaine, mepivacaine HCl, bupivacine HCl, pyrrocaine HCl, prilocaine HCl, digammacaine, and oxethazaine, or combination thereof.

Para-aminobenzoic acid esters are at least one of butacaine, dimethocaine, and tutocaine, or combination thereof.

Amino ethers are at least one of quinisocaine and pramocaine, or combination thereof.

A pharmaceutically acceptable aqueous solution comprises an antioxidant.

An antioxidant is at least one of beta-carotene, lutein, lycopene, bilirubin, vitamin A, vitamin C (ascorbic acid), vitamin E, uric acid, nitric oxide, nitroxide, pyruvate, catalase, superoxide dismutase, glutathione peroxidases, N-acetyl cysteine, and naringenin, or combination thereof.

A method for reducing the amount of localized subcutaneous fat of a subject, comprising a step of administering a pharmaceutical composition to the local site of the subject, wherein the pharmaceutical composition comprising: drug-containing micelles; and
resveratrol encapsulated in said drug-containing micelles;
wherein the drug-containing micelle is a microstructure formed by a pharmaceutically acceptable surfactant, and the hydrophilic-lipophilic balance value (HLB value) of the surfactant is greater than 10.

A diameter of the drug-containing micelles is 3~250 nm.

A diameter of the drug-containing micelles is 5~50 nm.

A surfactant is a non-ionic surfactant.

A non-ionic surfactant is at least one of polysorbate 80 (tween 80), solutol HS 15 (polyoxyl 15 hydroxystearate, 2-hydroxyethyl 12-hydroxyoctadecanoate), polyoxyethylene castor oils, and other non-ionic surfactants, or combination thereof.

Polyoxyethylene castor oils are at least one of cremophor ELP, cremophor RH 40, and other polyoxyethylene castor oils, or combination thereof.

A weight ratio of resveratrol to the surfactant is 1:4 to 1:500.

A weight ratio of resveratrol to the surfactant is 1:5 to 1:200.

A weight ratio of resveratrol to the surfactant is 1:8 to 1:80.

A concentration of resveratrol in the pharmaceutical composition is 0.2~ 166.7 mg/mL.

A concentration of resveratrol in the pharmaceutical composition is 2.5~ 60 mg/mL.

A pharmaceutical composition further comprises a pharmaceutically acceptable aqueous solution and a second lipophilic drug-containing micelle, wherein the second lipophilic drug-containing micelles are evenly distributed in the pharmaceutically acceptable aqueous solution; the second lipophilic drug-containing micelle is another microstructure formed by a second non-ionic surfactant, and a second lipophilic drug is encapsulated in said second lipophilic drug-containing micelle.

A hydrophilic-lipophilic balance value (HLB value) of the second non-ionic surfactant is greater than 10.

A second non-ionic surfactant is at least one of polysorbate 80 (tween 80), solutol HS 15 (polyoxyl 15 hydroxystearate, 2-hydroxyethyl 12-hydroxyoctadecanoate), polyoxyethylene castor oils, and other non-ionic surfactants, or combination thereof.

A second non-ionic surfactant is at least one of cremophor ELP, cremophor RH 40, and other polyoxyethylene castor oils, or combination thereof.

A second lipophilic drug is at least one of quercetin, synephrine, puerarin, curcuminoid, curcumin, and other lipophilic drugs except resveratrol, or combination thereof.

A weight ratio of resveratrol to the second lipophilic drug is 30:1 to 1:20.

A weight ratio of resveratrol to the second lipophilic drug is 20:1 to 1:15.

A weight ratio of resveratrol to the second lipophilic drug is 15:1 to 1:10.

A pharmaceutical composition further comprises a pharmaceutically acceptable aqueous solution and a hydrophilic drug.

A hydrophilic drug is at least one of green tea extract, epigallocatechin gallate, epicatechin, epicatechin gallate, epigallocatechin, gallocatechin gallate, gallocatechin, catechin gallate, catechin, epigallocatechin gallate (EGCG), caffeine, carnitine, L-carnitine, synephrine, chlorogenic acid, and other hydrophilic drugs, or combination thereof.

A weight ratio of resveratrol to the hydrophilic drugs is 20:1 to 1:30.

A weight ratio of resveratrol to the hydrophilic drugs is 15:1 to 1:20.

A weight ratio of resveratrol to the hydrophilic drugs is 10:1 to 1:15.

A hydrophilic drug is gallocatechin gallate, and the concentration of gallocatechin gallate in the pharmaceutical composition is 0.25~ 300 mg/mL.

A hydrophilic drug is gallocatechin gallate, and the concentration of gallocatechin gallate in the pharmaceutical composition is 1~ 200 mg/mL.

A hydrophilic drug is green tea extract, and the weight ratio of resveratrol to the green tea extract is 30:1 to 1:30.

A hydrophilic drug is green tea extract, and the weight ratio of resveratrol to the green tea extract is 20:1 to 1:20.

A hydrophilic drug is green tea extract, and the weight ratio of resveratrol to the green tea extract is 10:1 to 1:10.

An amount of the surfactant is 0.24 ~ 70 parts by weight, based on 1 part by weight of the total amount of resveratrol and green tea extract; or, the weight ratio of the total weight of the resveratrol and the green tea extract to the surfactant is 4:1 to 1:70.

An administered dosage of the pharmaceutical composition to be injected at the local site is 0.2 ~ 20 mg/cm².

An administered dosage of the pharmaceutical composition to be injected at the local site is 0.4 ~ 12 mg/cm².

An administered dosage of the pharmaceutical composition to be administered at the local site is 0.2 ~ 40 mg/kg.

An administered dosage of the pharmaceutical composition to be administered at the local site is 0.4 ~ 20 mg/kg.

A dosing frequency of the pharmaceutical composition is 1 to 12 times every other day to every 30 days.

A dosing frequency of the pharmaceutical composition is 1 to 8 times every other day to every 21 days.

S subject is an animal or a human.

A step is to inject or apply the pharmaceutical composition to the local site of the subject.

A pharmaceutical composition further comprises at least one of a cosolvent, a suspending agent, and an oil phase excipient, or combination thereof.

A microstructure is co-formed by the non-ionic surfactant and the oil phase excipient and/or the cosolvent.

A use of a pharmaceutical composition in preparing a drug or a subcutaneous injection formulation for reducing the amount of localized subcutaneous fat of a subject; the pharmaceutical composition comprising: drug-containing micelles; and
resveratrol encapsulated in said drug-containing micelles;
wherein the drug-containing micelle is a microstructure formed by a pharmaceutically acceptable non-ionic surfactant, and the hydrophilic-lipophilic balance value (HLB value) of the non-ionic surfactant is greater than 10.

A diameter of the drug-containing micelles is 3~250 nm.

A diameter of the drug-containing micelles is 5~50 nm.

A non-ionic surfactant is at least one of polysorbate 80 (tween 80), solutol HS 15 (polyoxyl 15 hydroxystearate, 2-hydroxyethyl 12-hydroxyoctadecanoate), polyoxyethylene castor oils, and other non-ionic surfactants, or combination thereof.

Polyoxyethylene castor oils are at least one of cremophor ELP, cremophor RH 40, and other polyoxyethylene castor oils, or combination thereof.

A weight ratio of resveratrol to the non-ionic surfactant is 1:4 to 1:500.

A concentration of resveratrol in the pharmaceutical composition is 0.2~ 166.7 mg/mL.

A pharmaceutical composition further comprises a pharmaceutically acceptable aqueous solution and a second lipophilic drug-containing micelle, wherein the second lipophilic drug-containing micelles are evenly distributed in the pharmaceutically acceptable aqueous solution; the second lipophilic drug-containing micelle is another microstructure formed by a second non-ionic surfactant, and a second lipophilic drug is encapsulated in said second lipophilic drug-containing micelle.

A hydrophilic-lipophilic balance value (HLB value) of the second non-ionic surfactant is greater than 10.

A second non-ionic surfactant is at least one of polysorbate 80 (tween 80), solutol HS 15 (polyoxyl 15 hydroxystearate, 2-hydroxyethyl 12-hydroxyoctadecanoate), polyoxyethylene castor oils, and other non-ionic surfactants, or combination thereof.

A second non-ionic surfactant is at least one of cremophor ELP, cremophor RH 40, and other polyoxyethylene castor oils, or combination thereof.

A second lipophilic drug is at least one of quercetin, synephrine, puerarin, curcuminoid, and other lipophilic drugs except resveratrol, or combination thereof.

A weight ratio of resveratrol to the second lipophilic drug is 30:1 to 1:20.

A pharmaceutical composition further comprises a pharmaceutically acceptable aqueous solution and a hydrophilic drug.

A hydrophilic drug is at least one of green tea extract, epigallocatechin gallate, epicatechin, epicatechin gallate, epigallocatechin, gallocatechin gallate, gallocatechin, catechin gallate, catechin, epigallocatechin gallate (EGCG), caffeine, carnitine, L-carnitine, synephrine, chlorogenic acid, and other hydrophilic drugs, or combination thereof.

A weight ratio of resveratrol to the hydrophilic drugs is 20:1 to 1:30.

A hydrophilic drug is gallocatechin gallate, and the concentration of gallocatechin gallate in the pharmaceutical composition for the formulation for subcutaneous fat layer injection or a formulation for subcutaneous injection is 0.25~ 300 mg/mL.

A hydrophilic drug is green tea extract, and the weight ratio of resveratrol to the green tea extract is 30:1 to 1:30.

An amount of the surfactant is 0.24 ~ 70 parts by weight, based on 1 part by weight of the total amount of resveratrol and green tea extract; or, the weight ratio of the total weight of the resveratrol and the green tea extract to the surfactant is 4:1 to 1:70.

A drug or subcutaneous injection formulation comprises a treatment effective amount of the pharmaceutical composition.

A treatment effective amount is to administrate 0.2 ~ 20 mg the pharmaceutical composition per cm² at the local site.

A treatment effective amount is to administrate 0.2 ~ 40 mg the pharmaceutical composition per kg of the body weight.

A dosing frequency of the drug or subcutaneous injection formulation is 1 to 12 times every other day to every 30 days.

A subject is an animal or a human.

A step is to inject the drug or subcutaneous injection formulation to the local site of the subject; or apply the drug to the local site of the subject.

A pharmaceutical composition further comprises at least one of a cosolvent, a suspending agent, and an oil phase excipient, or combination thereof.

A microstructure is co-formed by the non-ionic surfactant and the oil phase excipient and/or the cosolvent.

### [Experiments]

### Experiment 1: The effects of a resveratrol subcutaneous injection and a green tea extract subcutaneous injection on the subcutaneous fat and body weight of rats

Preparation of a green tea extract solution for subcutaneous injection:
a 5 mg/mL green tea extract aqueous solution was prepared with water for injection and green tea extract. The green tea extract aqueous solution was filtered through a 0.2 µm filter to obtain the 5 mg/mL green tea extract solution for subcutaneous injection in the present experiment. The green tea extract solution for subcutaneous injection had to be stored in dark at 4°C.

Preparation of a resveratrol solution for subcutaneous injection: a 5 mg/mL resveratrol solution was prepared with polysorbate 80(tween 80), which is an excipient commonly used by prior arts for non-hydrophilic drug formulations for injection, ethanol, water for injection, and resveratrol. The detailed preparation method is as follows: 0.5 g of resveratrol was mixed with an appropriate amount of ethanol. After resveratrol was completely dissolved, 0.1 g of polysorbate 80 (tween 80) was added to dissolve polysorbate 80 (tween 80) completely; the solution was vacuum-dried for 2 ~4 hours to volatilize ethanol; once ethanol was completely volatilized, the total volume was adjusted to 100 mL by adding water for injection; the solution was stirred well and filtered through a 0.2 µm filter to obtain the 5 mg/mL resveratrol solution for subcutaneous injection in the present experiment. The resveratrol solution for subcutaneous injection had to be stored in dark at 4°C.

Seven-week-old male Sprague-Dawley rats were used for the experiment. First, 18 rats were fed with high-fat diet (brand name Research Diets, Inc; catalog number D12492) to induce the accumulation of the subcutaneous fat until each rat weighed 330 ±10 g. The rats were randomly assigned into three groups: a high-fat diet control group, a resveratrol group, and a green tea extract group, with 6 rats in each group such that there was no statistical difference in the body weight between groups. The body weight of each rat was recorded and defined as the "pre-experimental body weight". Then, drugs were administrated as follows:

The 5 mg/mL resveratrol solution for subcutaneous injection was injected into the lower inguinal subcutaneous fat layer of rats in the resveratrol group. Each injected dosage was 8 mg of resveratrol per kilogram (8 mg/kg) of body weight; that is, 1.6 mL of the aforementioned 5 mg/mL resveratrol solution for subcutaneous injection was injected per kilogram of body weight. The 5 mg/mL green tea extract solution for subcutaneous injection was injected into the lower inguinal subcutaneous fat layer of rats in the green tea extract group. Each injected dosage was 8 mg of green tea extract per kilogram (8 mg/kg) of body weight; that is, 1.6 mL of the aforementioned 5 mg/mL green tea extract solution for subcutaneous injection was injected per kilogram of body weight. Rats in the high-fat diet control group were injected with the same volume of water for injection in the same manner described above.

The aforementioned injection sites were the lower inguinal fat pads of rats. Bilateral injections were performed evenly at the injection sites once a day on day 1, 3, and 5 of the experiment. The rats were fed with high-fat diet during the entire duration of the experiment. Their weight change was recorded daily, and their water and food consumption was recorded weekly. Rats were fasted on day 20 and euthanized by CO₂ on day 21.

The body weight of each rat was recorded and defined as its "post-experimental body weight". The "total body weight gain" of each rat was obtained by subtracting its "pre-experimental body weight" from its "post-experimental body weight". The "relative weight gain" was obtained by dividing the total body weight gain of each group by the total body weight gain of the high-fat diet control group.

The bilateral inguinal fat pads of rats were dissected and weighed, and the amount of the lower inguinal fat of each group was calculated. The data were presented as mean ± SD and analyzed by one-way ANOVA. Statistical analysis results were presented as symbols or letters. Different symbols or letters indicate statistical difference (p<0.05), and identical symbols or letters indicate no statistical difference (p>0.05).

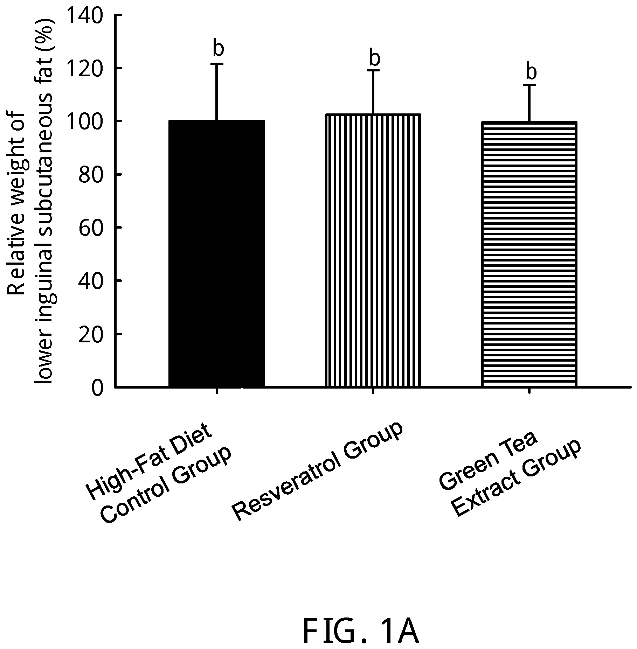

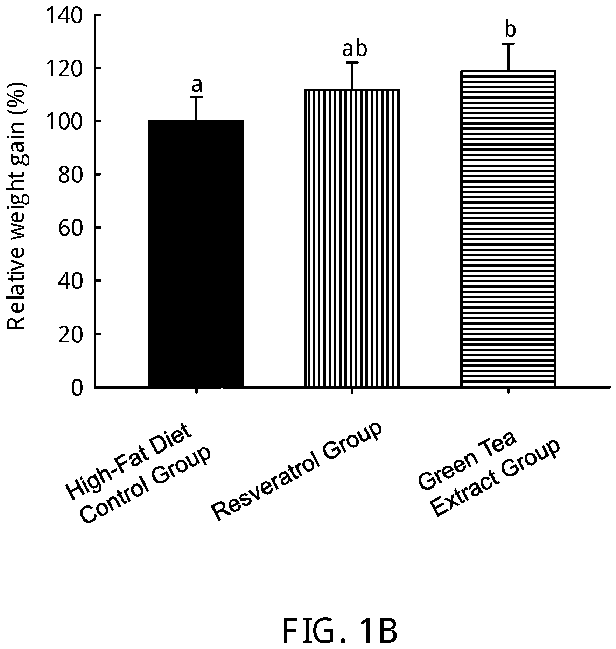

Please refer to Figure 1A and 1B. Figure 1A is a bar graph showing the effects of the non-micellar resveratrol solution for subcutaneous injection and the non-micellar green tea extract solution for subcutaneous injection on the relative weight of lower inguinal subcutaneous fat of rats. Figure 1B is a bar graph showing the effects of the non-micellar resveratrol solution for subcutaneous injection and the non-micellar green tea extract solution for subcutaneous injection on the relative weight gain of rats. Wherein, said amount of lower inguinal fat is the total amount of bilateral lower inguinal fat.

In Figure 1A, the results showed that after three times of subcutaneous injections to deliver the drugs, comparing to the high-fat diet control group, there was no significant reduction in the localized fat at the injection site of rats in the resveratrol group or the green tea extract group (p>0.05). This indicates that direct injection of dissolved resveratrol or green tea extract to subcutaneous fat layer cannot reduce the localized fat at the injection site.

In Figure 1B, the results showed that after three times of subcutaneous injections to deliver the drugs, comparing to the high-fat diet control group, there was no significant reduction in the body weight of rats in the resveratrol group or the green tea extract group (p>0.05). This indicates that direct injection of dissolved resveratrol or green tea extract to subcutaneous fat layer cannot reduce the body weight.

Wherein, green tea extract is an ingredient with very high solubility in water, and previous studies have shown that it promotes adipocyte apoptosis in cell-based experiments. However, direct injection of dissolved green tea extract to the subcutaneous fat layer surprisingly can reduce neither the localized subcutaneous fat nor the body weight. This experiment demonstrated that direct injection of dissolved resveratrol or green tea extract to the subcutaneous fat layer cannot reduce the localized fat and body weight. In order to overcome this problem, the inventor conducted further studies to develop the resveratrol-containing pharmaceutical composition in the present invention.

### Experiment 2: The effects of different kinds of resveratrol subcutaneous injection formulation on the subcutaneous fat and body weight of rats

Resveratrol normal saline solution, resveratrol PEG solution, and resveratrol ELP solution were prepared as follows:

### Preparation of the resveratrol normal saline solution:

500 mg of resveratrol was mixed with an appropriate amount of normal saline for injection to make the total volume become 100 mL. The solution was stirred well to completely dissolve resveratrol to obtain the resveratrol normal saline solution. The concentration of resveratrol in said resveratrol normal saline solution was 5 mg/mL.

### Preparation of the resveratrol PEG solution.

15g of polyethylene glycol 400 (abbreviated as PEG 400) and 15 g of glycerol were mixed with an appropriate amount of normal saline for injection to make the total volume become 100 mL. The solution was stirred well to completely dissolve PEG 400 and glycerol to obtain a PEG-glycerol mixture. 450 mg of resveratrol was mixed with an appropriate amount of the PEG-glycerol mixture to make the total volume become 90 mL. The solution was stirred well to completely dissolve resveratrol to obtain the resveratrol PEG solution. The concentration of resveratrol in said resveratrol PEG solution was 5 mg/mL.

### Preparation of the resveratrol ELP solution:

500 mg of resveratrol was mixed with 100~160 mL of dichloromethane, and stirred at 150~500 rpm at room temperature until resveratrol dissolved completely. 20 g of Kolliphor ELP (also known as polyoxyl 35 castor oil, polyoxyl 35 hydrogenated castor oil, or Cremophor ELP abbreviated as ELP) was added to the solution and stirred well at 100~300 rpm to volatize dichloromethane. Once dichloromethane volatized completely, normal saline for injection was slowly added to make the total volume become 100 mL, and the solution was stirred well to obtain the resveratrol ELP solution. In said resveratrol ELP solution, the concentration of resveratrol was 5 mg/mL, the concentration of ELP was approximately 20% (wt.%), and the weight ratio of resveratrol to ELP was 1:40.

Six-week-old male Sprague-Dawley rats were used for the experiment. First, 20 rats were fed with high-fat diet (Research Diets, Inc.; Cat #D12492) to induce the accumulation of subcutaneous fat. Feeding was continued until each rat weighed 330±10 g, and the rats were randomly assigned into four groups: a control group, a normal saline group, a PEG group, and an ELP group, with 5 rats in each group such that there was no statistical difference in the body weight between groups. The body weight of each rat was recorded and defined as the "pre-experimental body weight". Then, drugs were administrated as follows:
The resveratrol normal saline solution, the resveratrol PEG solution, and the resveratrol ELP solution were injected to the lower inguinal subcutaneous fat pads of rats in the normal saline group, the PEG group, and the ELP group, respectively. Each injected dosage was 4 mL per kilogram of body weight (4 mL/kg), such that each injected dosage was 20 mg of resveratrol per kilogram of body weight (20 mg/kg; 4 mL/kg x 5 mg/mL = 20 mg/kg). Rats in the control group were injected with the same volume of normal saline in the same manner mentioned above.

The injection sites mentioned above were the lower inguinal fat pads of rats. Bilateral injections were administrated evenly once a day on day 1, 2, 3, and 4 of the experiment. The rats were fed with high-fat diet for the entire duration of the experiment. Their weight changes were recorded daily, and food and water consumption was recorded weekly. The experiment lasted for 14 days, and the rats were euthanized by CO₂ on day 15.

The body weight of each rat was recorded and defined as its "post-experimental body weight". The "total body weight gain" of each rat was obtained by subtracting its "pre-experimental body weight" from its "post-experimental body weight". The "relative weight gain" was obtained by dividing the total body weight gain of each group by the total body weight gain of the control group.

The bilateral lower inguinal subcutaneous fat pads of rats were dissected and weighed, and the weights of the bilateral lower inguinal subcutaneous fat pads were summed to calculate the amount of lower inguinal subcutaneous fat. The amount of lower inguinal subcutaneous fat of each group was divided by the amount of lower inguinal subcutaneous fat of the control group to obtain the "relative weight of lower inguinal subcutaneous fat".

The data were presented as mean ± SD and analyzed by one-way ANOVA. Statistical results were shown as symbols or letters. Different symbols or letters indicated statistical difference (p<0.05), and identical symbols or letters indicated no statistical difference (p>0.05).

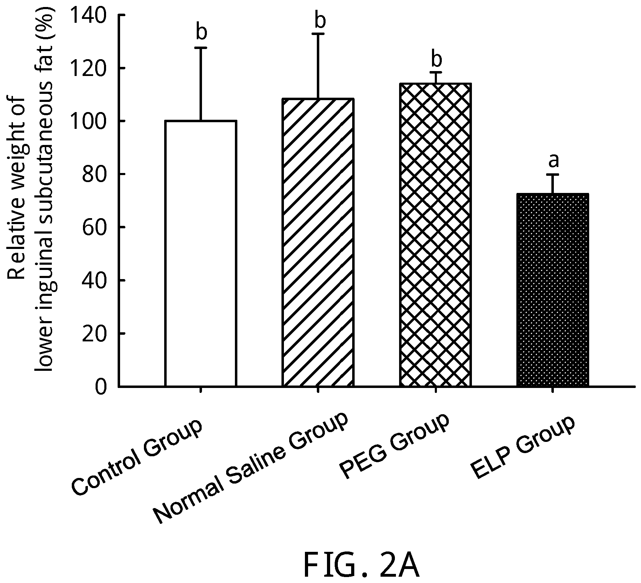

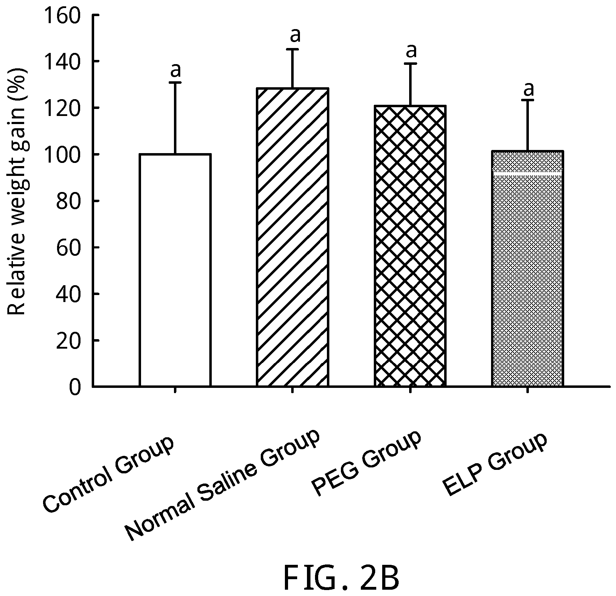

Please refer to Figure 2A and Figure 2B. Figure 2A is a bar graph showing the effects of resveratrol subcutaneous injection formulations prepared with different excipients on the amount of localized subcutaneous fat of rats. Figure 2B is a bar graph showing the effect of resveratrol subcutaneous injection formulations prepared with different excipients on the relative weight gain of rats

As shown in Figure 2A, the relative weight of lower inguinal subcutaneous fat of rats in the control group was 100±27.6%, the relative weight of lower inguinal subcutaneous fat of rats in the normal saline group was 108.2±24.7%, the relative weight of lower inguinal subcutaneous fat of rats in the PEG group was 114.0±4.4%, and the relative weight of lower inguinal subcutaneous fat of rats in the ELP group was 72.5±0.0%. There was no significant difference in the relative weight of lower inguinal subcutaneous fat between the normal saline and the control group, suggesting that direct injection of resveratrol to the subcutaneous fat layer of the administration site cannot reduce the fat at the administration site (the localized fat). There was no significant difference in the relative weight of lower inguinal subcutaneous fat between the PEG group and the control group; however, the relative weight of lower inguinal subcutaneous fat of the ELP group was significantly different (p<0.05) from that of the control group, and the relative weight of lower inguinal subcutaneous fat of rats in the ELP group was reduced by 27.5%.

As shown in Figure 2B, the relative weight gain of rats in the control group was 100.0±30.8%, the relative weight gain of rats in the normal saline group was 128.3±16.9%, the relative weight gain of rats in the PEG group is 120.8±18.2%, and the relative weight gain of rats in the ELP group was 101.3±22.0%. There was no significant difference between the four groups (p>0.05).

The above experiments demonstrated that direct injection of resveratrol to the subcutaneous fat layer of the administration site cannot reduce the fat at the administration site (the localized fat), nor can it reduce body weight. Direct injection of the resveratrol composition comprising the excipient PEG (a commonly used cosolvent) to the subcutaneous fat layer of the administration site cannot reduce the fat at the administration site (the localized fat), nor can reduce body weight; but, injection of the resveratrol composition comprising the non-ionic surfactant ELP to the subcutaneous fat layer of the administration site can reduce the fat at the administration site (the localized fat) significantly. Thus, it is necessary to investigate further that whether the resveratrol mixture has to comprise non-ionic surfactants so that the fat at the administration site (the localized fat) and body weight can be reduced.

Further analysis showed that there were no micelles in the aforementioned resveratrol PEG solution, but there were micelles in the resveratrol ELP solution, and resveratrol was encapsulated in the micelles formed by ELP. Thus, it is necessary to further investigate the effect of micelles on reducing localized fat and reducing body weight.

### Experiment 3: The effects of resveratrol simple composition subcutaneous injection formulation comprising micelles on the amount of subcutaneous fat and body weight of rats

Resveratrol ELP partial micellar formulation, resveratrol HS-15 partial micellar formulation, resveratrol ELP micellar formulation, and resveratrol HS-15 micellar formulation were prepared as follows:
Preparation of the resveratrol ELP partial micellar formulation: 20 g of Kolliphor ELP (also known as polyoxyl 35 castor oil, polyoxyl 35 hydrogenated castor oil, or Cremophor ELP, abbreviated as ELP) was mixed with an appropriate amount of normal saline for injection to make the total weight become 100 g. The solution was stirred well to completely dissolve Kolliphor ELP (also known as ELP) to obtain a 20% ELP solution. 400 mg of resveratrol was mixed with an appropriate amount of the 20% ELP solution to make the total weight become 80 g. The solution was stirred well to completely dissolve resveratrol to obtain the resveratrol ELP partial micellar formulation. In said resveratrol ELP partial micellar formulation, the concentration of resveratrol was approximately 5 mg/mL, the concentration of ELP was approximately 20% (wt.%), and the weight ratio of resveratrol to ELP was approximately 1:40.

Preparation of the resveratrol HS-15 partial micellar formulation: 20 g of Kolliphor HS-15 (abbreviated as HS-15) was mixed with an appropriate amount of normal saline for injection to make the total weight become 100 g. The solution was stirred well to completely dissolve HS-15 to obtain a 20% HS-15 solution. 400 mg of resveratrol was mixed with an appropriate amount of the 20% HS-15 solution to make the total weight become 80 g. The solution was stirred well to completely dissolve resveratrol to obtain the resveratrol HS-15 partial micellar formulation. in said resveratrol HS-15 partial micellar formulation, the concentration of resveratrol was approximately 5 mg/mL, the concentration of HS-15 was approximately 20% (wt.%), and the weight ratio of resveratrol to HS-15 was approximately 1:40.

Preparation of the resveratrol ELP micellar formulation: it is the same as the preparation method of the resveratrol ELP solution in Experiment 2.

Preparation of the resveratrol HS-15 micellar formulation: 500 mg of resveratrol was mixed with 80~140 mL of dichloromethane and stirred at 150~500 rpm at room temperature until resveratrol dissolved completely. 20 g of Kolliphor HS-15 (abbreviated as HS-15) was added and stirred at 100~300 rpm to volatize dichloromethane. Once dichloromethane volatized completely, normal saline for injection was slowly added to make the total weight become 100 g. The solution was stirred well to form drug-containing micelles to obtain the resveratrol HS-15 micellar formulation. In said resveratrol HS-15 micellar formulation, the concentration of resveratrol was approximately 5 mg/g, the concentration of HS-15 was approximately 20% (wt.%), and the weight ratio of resveratrol to HS-15 was approximately 1:40.

The resveratrol ELP partial micellar formulation, the resveratrol HS-15 partial micellar formulation, the resveratrol ELP micellar formulation, and the resveratrol HS-15 micellar formulation were analyzed by a particle size analyzer to determine if micelles were present therein, and determine the diameter of micelles was measured.

The results showed that both the resveratrol ELP partial micellar formulation and the resveratrol HS-15 partial micellar formulation had large quantities of precipitates, and had a lower number of drug-containing micelles. However, the resveratrol ELP micellar formulation and the resveratrol HS-15 micellar formulation were clear without stratification, and had a higher number of drug-containing micelles.

The results indicated that although both of the resveratrol ELP partial micellar formulation and the resveratrol HS-15 partial micellar formulation both had large quantities of precipitates, their supernate still contained micelles. Therefore, the resveratrol ELP partial micellar formulation, the resveratrol HS-15 partial micellar formulation, the resveratrol ELP micellar formulation, and the resveratrol HS-15 micellar formulation are all pharmaceutical compositions of the present invention.

Six-week-old male Sprague-Dawley rats were used for the experiment. First, 20 rats were fed with high-fat diet (Research Diets, Inc.; Cat #D12492) to induce the accumulation of subcutaneous fat. Feeding was continued until each rat weighed 330±10 g, and the rats were randomly assigned into 5 groups: a control group, an ELP partial micellar group, an HS-15 partial micellar group, an ELP micellar group, and an HS-15 micellar group, with 4 rats in each group such that there was no statistical difference in the body weight between groups. The body weight of each rat was recorded and defined as the "pre-experimental body weight". Then, drugs were administrated as follows:

The resveratrol ELP partial micellar formulation, the resveratrol HS-15 partial micellar formulation, the resveratrol ELP micellar formulation, and the resveratrol HS-15 micellar formulation were each mixed well (to evenly suspend the precipitates in the partial micellar formulations), and were injected into the lower inguinal subcutaneous fat layer of rats in the ELP partial micellar group, the HS-15 partial micellar group, the ELP micellar group, and the HS-15 micellar group, respectively. Each injected dosage was 4 mL per kilogram of body weight (4 mL/kg), such that each injected dosage was 20 mg of resveratrol per kilogram of body weight (20 mg/kg; the calculation is as follows 4mL/kg x 5 mg/mL = 20 mg/kg). Rats in the control group were injected with the same volume of normal saline in the same manner mentioned above.

The injection sites mentioned above were the lower inguinal fat pads of rats. Bilateral injections were administrated evenly once a day on day 1, 2, 3, 4, 5, and 6 of the experiment. The rats were fed with high-fat diet for the entire duration of the experiment. Their weight changes were recorded daily, and food and water consumption was recorded weekly. The experiment lasted for 14 days, and the rats were euthanized with CO₂ on day 15.

The body weight of each rat was recorded and defined as its "post-experimental body weight". The "total body weight gain" of each rat was obtained by subtracting its "pre-experimental body weight" from its "post-experimental body weight". The "relative weight gain" was obtained by dividing the total body weight gain of each group by the total body weight gain of the control group.

The bilateral lower inguinal subcutaneous fat pads of rats were dissected and weighed, and the weights of the bilateral lower inguinal subcutaneous fat pads were summed to calculate the amount of lower inguinal subcutaneous fat. The amount of lower inguinal subcutaneous fat of each group was divided by the amount of lower inguinal subcutaneous fat of the control group to obtain the "relative weight of lower inguinal subcutaneous fat".

The data were presented as mean ± SD and analyzed by one-way ANOVA. Statistical results were shown as symbols or letters. Different symbols or letters indicated statistical difference (p<0.05), and identical symbols or letters indicated no statistical difference (p>0.05).

Based on the formulation preparation methods and the result of particle size analyses above, the concentration of ELP and the concentration of resveratrol in the resveratrol ELP partial micellar formulation were identical to those of the resveratrol ELP micellar formulation, only the number of drug-containing micelles differed. Thus, comparing to the resveratrol ELP partial micellar formulation, if the resveratrol ELP micellar formulation can significantly reduce the localized fat at the administration site, this indicates that formation of drug-containing micelles is the critical factor of resveratrol compositions to significantly reduce the localized fat at the administration site; if the resveratrol ELP micellar formulation can significantly reduce the body weight, this indicates that formation of drug-containing micelles is the critical factor of resveratrol compositions to significantly reduce the body weight.

Similarly, the concentration of HS-15 and the concentration of resveratrol in the resveratrol HS-15 partial micellar formulation were identical to those of the resveratrol HS-15 micellar formulation, only the number of drug-containing micelles differed. Thus, comparing to the resveratrol HS-15 partial micellar formulation, if the resveratrol HS-15 micellar formulation can significantly reduce the localized fat at the administration site, this indicates that formation of drug-containing micelles is the critical factor of resveratrol compositions to significantly reduce the localized fat at the administration site; if the resveratrol HS-15 micellar formulation can significantly reduce the body weight, this indicates that formation of drug-containing micelles is the critical factor of resveratrol compositions to significantly reduce the body weight.

The results showed that injection of resveratrol ELP partial micellar formulation or resveratrol HS-15 partial micellar formulation to the subcutaneous fat layer of the administration site can reduce the fat at the administration site (the localized fat). On the other hand, injection of resveratrol ELP micellar formulation or resveratrol HS-15 micellar formulation to the subcutaneous fat layer of the administration site can also reduce the fat at the administration site (the localized fat). Between the four formulations, resveratrol ELP micellar formulation has the best effect on reducing localized fat.

Comparing to the resveratrol ELP partial micellar formulation, the resveratrol ELP micellar formulation can reduce the localized fat significantly. Comparing to resveratrol HS-15 partial micellar formulation, resveratrol HS-15 micellar formulation can reduce the localized fat significantly.

The results indicated that formation of micelles is the critical factor of resveratrol compositions to significantly reduce the localized fat at the administration site, and the drug-containing micelles formed by polyoxyethylene castor oil (such as ELP) has the best effect on reducing localized fat.

The results showed that injection of resveratrol ELP partial micellar formulation or resveratrol HS-15 partial micellar formulation to the subcutaneous fat layer of the administration site can reduce the body weight. On the other hand, injection of resveratrol ELP micellar formulation or resveratrol HS-15 micellar formulation to the subcutaneous fat layer of the administration site can also reduce the body weight. Between the four formulations, resveratrol ELP micellar formulation has the best effect on reducing body weight.

This experiment indicated that formation of micelles is the critical factor of resveratrol compositions to significantly reduce the body weight, and the drug-containing micelles formed by polyoxyethylene castor oil (such as ELP) has the best effect on reducing body weight.

### Experiment 4: Preparation of pharmaceutical compositions for reducing localized fat

This experiment prepared a first pharmaceutical composition with resveratrol, and prepared a second pharmaceutical composition with resveratrol and green tea extract.

Preparation of the first pharmaceutical composition were as follows:
(a) Mixing a first weight of resveratrol with a solvent and stirring at 200 ~ 500 rpm at room temperature until resveratrol is completely dissolved;
(b) Adding a second weight of a pharmaceutically acceptable surfactant and stirring well at 100 ~ 300 rpm to volatilize the solvent, wherein the hydrophilic-lipophilic balance value (HLB value) of the surfactant is greater than 10;
(c) Once the solvent volatilized completely, slowly adding a third weight of a pharmaceutically acceptable aqueous solution and stirring well to obtain drug-containing micelles; and
(d) Filtering through a 0.2 µm filter and storing the filtered solution comprising drug-containing micelles in dark and refrigeration;

Wherein, in step (c), the drug-containing micelle is a microstructure formed by the surfactant, and resveratrol is encapsulated in said drug-containing micelle.

Preferably, the third weight is heavier than or equal to 0 g.

Preferably, in step (a), the boiling point of the solvent is lower than that of pure water.

Preferably, in step (a), the solvent is a hydrophilic solvent.

Preferably, the hydrophilic solvent is at least one of methanol, ethanol, acetone, and other hydrophilic solvents, or combination thereof.

Preferably, the solvent in step (a) is a lipophilic solvent.

Preferably, the lipophilic solvent is at least one of ether, benzene, chloroform, ethyl acetate, dichloromethane, hexane, and other lipophilic solvents, or combination thereof.

Preferably, in step (b), the surfactant is a non-ionic surfactant.

Preferably, the non-ionic surfactant is at least one of polysorbate 80 (tween 80), solutol HS 15 (polyoxyl 15 hydroxystearate, 2-hydroxyethyl 12-hydroxyoctadecanoate), polyoxyethylene castor oils, and other non-ionic surfactants, or combination thereof.

Preferably, the polyoxyethylene castor oils are at least one of cremophor ELP, cremophor RH 40, and other polyoxyethylene castor oils, or combination thereof.

Preferably, in steps (a) and (b), the weight ratio of the first weight of resveratrol and the second weight of surfactant is 1:4 to 1:500.

Preferably, in steps (a) and (c), the weight ratio of the first weight of resveratrol and the third weight of pharmaceutically acceptable aqueous solution is 1:400 to 3:50.

Preferably, in step (c), the pharmaceutically acceptable aqueous solution is water for injection, aqueous solution for injection, or normal saline.

Preferably, in step (c), the pharmaceutically acceptable solution comprises a local anesthetic.

Preferably, the local anesthetic is at least one of amides, para-aminobenzoic acid esters, and amino ethers, or combination thereof.

Preferably, the amides are at least one of dibucaine, lidocaine, mepivacaine HCl, bupivacine HCl, pyrrocaine HCl, prilocaine HCl, digammacaine, and oxethazaine, or combination thereof.

Preferably, the para-aminobenzoic acid esters are at least one of butacaine, dimethocaine, and tutocaine, or combination thereof.

Preferably, the amino ethers are at least one of quinisocaine and pramocaine, or combination thereof.

Preferably, in step (c), the pharmaceutically acceptable aqueous solution comprises an antioxidant.

Preferably, the antioxidant is at least one of beta-carotene, lutein, lycopene, bilirubin, vitamin A, vitamin C (ascorbic acid), vitamin E, uric acid, nitric oxide, nitroxide, pyruvate, catalase, superoxide dismutase, glutathione peroxidases, N-acetyl cysteine, and naringenin, or combination thereof.

Preparation of the second pharmaceutical composition were as follows:
(a1) Mixing a fourth weight of resveratrol with a solvent, and stirring at 200 ~ 500 rpm at room temperature until resveratrol is completely dissolved;
(b1) Adding a fifth weight of a pharmaceutically acceptable surfactant and stirring well at 100 ~ 300 rpm to volatilize the solvent, wherein the hydrophilic-lipophilic balance value (HLB value) of the surfactant is greater than 10;
(c1) Once the solvent volatilized completely, slowly adding a sixth weight of a first pharmaceutically acceptable aqueous solution and stirring well at 100 ~ 300 rpm to form drug-containing micelles; and
(d1) Filtering through a 0.2 µm filter and storing the filtered solution comprising drug-containing micelles in dark and refrigeration;

Wherein, the first pharmaceutically acceptable aqueous solution comprises a seventh weight of green tea extract; the green tea extract comprises a first green tea extract ingredient, and said first green tea extract ingredient is epigallocatechin gallate.

Preferably, in step (a1), the boiling point of the solvent is lower than that of pure water.

Preferably, in step (a1), the solvent is a hydrophilic solvent.

Preferably, the hydrophilic solvent is at least one of methanol, ethanol, acetone, and other hydrophilic solvents, or combination thereof.

Preferably, the solvent in step (a1) is a lipophilic solvent.

Preferably, the lipophilic solvent is at least one of ether, benzene, chloroform, ethyl acetate, dichloromethane, hexane, and other lipophilic solvents, or combination thereof.

Preferably, in step (b1), the surfactant is a non-ionic surfactant.

Preferably, the non-ionic surfactant is at least one of polysorbate 80 (tween 80), solutol HS 15 (polyoxyl 15 hydroxystearate, 2-hydroxyethyl 12-hydroxyoctadecanoate), polyoxyethylene castor oils, and other non-ionic surfactants, or combination thereof.

Preferably, the polyoxyethylene castor oils are at least one of cremophor ELP, cremophor RH 40, and other polyoxyethylene castor oils, or combination thereof.

Preferably, between step (c1) and (d1), further comprising a step:
(c11) Adding an eighth weight of a second pharmaceutically acceptable aqueous solution and mixing well until the second pharmaceutically acceptable aqueous solution is completely dissolved.

Preferably, the green tea extract is dissolved in the first pharmaceutically acceptable aqueous solution, and the drug-containing micelle is a microstructure formed by the surfactant, wherein resveratrol is encapsulated in said drug-containing micelle.

Preferably, in the green tea extract dissolved in the first pharmaceutically acceptable aqueous solution, the content of epigallocatechin gallate is 45 ~100% by weight, based on 100% by weight of the total green tea extract.

Preferably, in steps (a1) and (c1), the weight ratio of the fourth weight of resveratrol and the seventh weight of green tea extract is 30:1 to 1:30.

Preferably, in steps (a1) to (c1), the amount of the fifth weight of surfactant is 0.24 ~ 70 parts by weight, based on 1 part by weight of the total amount of the fourth weight of resveratrol and the seventh weight of the green tea extract; or, the weight ratio of the total weight of the resveratrol and the green tea extract to the surfactant is 4:1 to 1:70.

Preferably, in steps (a1), (c1), and (c11), the total amount of the sixth weight of the first pharmaceutically acceptable aqueous solution and the eighth weight of the second pharmaceutically acceptable aqueous solution is 16 ~ 400 parts by weight, based on 1 part by weight of the total amount of the fourth weight of resveratrol and the seventh weight of the green tea extract; or, the ratio of the sum of the fourth weight and the seventh weight to the sum of the sixth weight and the eighth weight is 1:400 ~ 3:50.
the total amount of the first pharmaceutically acceptable aqueous solution and the second pharmaceutically acceptable aqueous solution is 10 ~1000 parts by weight, based on 1 part by weight of the total amount of resveratrol and green tea extract; or, the ratio of the total weight of the resveratrol and the green tea extract to the total weight of the first pharmaceutically acceptable aqueous solution and the second pharmaceutically acceptable solution is 1:1000 ~ 1:10.

Preferably, in steps (c1) and (c11), the first pharmaceutically acceptable aqueous solution and the second pharmaceutically acceptable aqueous solution are water for injection, aqueous solution for injection, or normal saline.

Preferably, in step (c1), the first pharmaceutically acceptable aqueous solution comprises a local anesthetic.

Preferably, the local anesthetic is at least one of amides, para-aminobenzoic acid esters, and amino ethers, or combination thereof.

Preferably, the amides are at least one of dibucaine, lidocaine, mepivacaine HCl, bupivacine HCl, pyrrocaine HCl, prilocaine HCl, digammacaine, and oxethazaine, or combination thereof
Preferably, the para-aminobenzoic acid esters are at least one of butacaine, dimethocaine, and tutocaine, or combination thereof.

Preferably, the amino ethers are at least one of quinisocaine and pramocaine, or combination thereof.

Preferably, in step (c1), the first pharmaceutically acceptable aqueous solution comprises an antioxidant.

Preferably, the antioxidant is at least one of beta-carotene, lutein, lycopene, bilirubin, vitamin A, vitamin C (ascorbic acid), vitamin E, uric acid, nitric oxide, nitroxide, pyruvate, catalase, superoxide dismutase, glutathione peroxidases, N-acetyl cysteine, and naringenin, or combination thereof.

This experiment prepared a third pharmaceutical composition with resveratrol and a hydrophilic drug, and prepared a fourth pharmaceutical composition and a fifth pharmaceutical composition with resveratrol and other lipophilic drugs.

Preparation of the third pharmaceutical composition were as follows:
(a2) Mixing resveratrol with a solvent and stirring at room temperature at 200 ~ 500 rpm until resveratrol is completely dissolved;
(b2) Adding a pharmaceutically acceptable surfactant and stirring well at 100 ~ 300 rpm to volatilize the solvent, wherein the hydrophilic-lipophilic balance value (HLB value) of the surfactant is greater than 10;
(c2) Once the solvent volatilized completely, slowly adding the first pharmaceutically acceptable aqueous solution and stirring well at 100 ~ 300 rpm to form drug-containing micelles; and
(d2) Filtering through a 0.2 µm filter and storing the filtered solution comprising drug-containing micelles in dark and refrigeration;

Wherein, the first pharmaceutically acceptable aqueous solution comprises a hydrophilic drug.

Preferably, the first pharmaceutically acceptable aqueous solution comprises a local anesthetic.

Preferably, the local anesthetic is at least one of amides, para-aminobenzoic acid esters, and amino ethers, or combination thereof.

Preferably, the amides are at least one of dibucaine, lidocaine, mepivacaine HCl, bupivacine HCl, pyrrocaine HCl, prilocaine HCl, digammacaine, and oxethazaine, or combination thereof.

Preferably, the para-aminobenzoic acid esters are at least one of butacaine, dimethocaine, and tutocaine, or combination thereof.

Preferably, the amino ethers are at least one of quinisocaine and pramocaine, or combination thereof.

Preferably, the first pharmaceutically acceptable aqueous solution comprises an antioxidant.

Preferably, the antioxidant is at least one of beta-carotene, lutein, lycopene, bilirubin, vitamin A, vitamin C (ascorbic acid), vitamin E, uric acid, nitric oxide, nitroxide, pyruvate, catalase, superoxide dismutase, glutathione peroxidases, N-acetyl cysteine, and naringenin, or combination thereof.

Preferably, in step (a2), the boiling point of the solvent is lower than that of pure water.

Preferably, in step (a2), the solvent is a hydrophilic solvent.

Preferably, the hydrophilic solvent is at least one of methanol, ethanol, acetone, and other hydrophilic solvents, or combination thereof.

Preferably, the solvent in step (a2) is a lipophilic solvent.

Preferably, the lipophilic solvent is at least one of ether, benzene, chloroform, ethyl acetate, dichloromethane, hexane, and other lipophilic solvents, or combination thereof.

Preferably, in step (b2), the surfactant is a non-ionic surfactant.

Preferably, the non-ionic surfactant is at least one of polysorbate 80 (tween 80), solutol HS 15 (polyoxyl 15 hydroxystearate, 2-hydroxyethyl 12-hydroxyoctadecanoate), polyoxyethylene castor oils, and other non-ionic surfactants, or combination thereof.

Preferably, the polyoxyethylene castor oils, are at least one of cremophor ELP, cremophor RH 40, and other polyoxyethylene castor oils, or combination thereof.

Preferably, between steps (c2) and (d2), further comprising a step:
(c21)Adding a second pharmaceutically acceptable aqueous solution and stirring well until the second pharmaceutically acceptable aqueous solution is completely dissolved.

Preferably, the hydrophilic drug is dissolved in the first pharmaceutically acceptable aqueous solution, and the drug-containing micelle is a microstructure formed by the surfactant, and resveratrol is encapsulated in said drug-containing micelle.

Preferably, the hydrophilic drug in the first pharmaceutically acceptable aqueous solution is at least one of green tea extract, epigallocatechin gallate, epicatechin, epicatechin gallate, epigallocatechin, gallocatechin gallate, gallocatechin, catechin gallate, catechin, epigallocatechin gallate (EGCG), caffeine, carnitine, L-carnitine, synephrine, chlorogenic acid, and other hydrophilic drugs, or combination thereof.

Preferably, in steps (a2) and (c2), the weight ratio of the resveratrol to the hydrophilic drug is 30:1 to 1:30.

Preferably, in steps (a2) ~ (c2), the amount of the surfactant is 0.24 ~ 70 parts by weight, based on 1 part by weight of the total amount of resveratrol and the hydrophilic drug; or, the weight ratio of the total weight of the resveratrol and the hydrophilic drug to the surfactant is 4:1 to 1:70.

Preferably, in step (a2), (c2), and (c21), using the total weight of resveratrol and the hydrophilic drug as a weight unit, the total amount of the first pharmaceutically acceptable aqueous solution and the second pharmaceutically acceptable solution is 16 ~ 400 parts by weight, based on 1 part by weight of the total amount of resveratrol and the hydrophilic drug; or, the ratio of the sum of the fourth weight and the seventh weight to the sum of the sixth weight and the eighth weight is 1:400 ~ 3:50.

Preferably, in steps (c2) and (c21), the first pharmaceutically acceptable aqueous solution and the second pharmaceutically acceptable aqueous solution are water for injection, aqueous solution for injection, or normal saline.

Preparation of the fourth pharmaceutical composition were as follows:
(A) A step of preparation of a drug-containing micelle subassembly, for preparing a drug-containing micelle subassembly;
(B) A step of preparation of a second lipophilic drug-containing micelle subassembly, for preparing a second lipophilic drug-containing micelle subassembly; and
(C) Mixing the drug-containing micelle subassembly with the second lipophilic drug-containing micelle subassembly to prepare the fourth pharmaceutical composition;
   Wherein, the step (A) for preparing the drug-containing micelle subassembly comprises the following steps (a3) ~ (d3):
      (a3) Mixing resveratrol with a first solvent and stirring at 200 ~ 500 rpm at room temperature until resveratrol is completely dissolved;
      (b3) Adding a first pharmaceutically acceptable surfactant and stirring well at 100 ~ 300 rpm to volatilize the first solvent, wherein the hydrophilic-lipophilic balance value (HLB value) of the first surfactant is greater than 10;
      (c3) Once the first solvent volatilized completely, slowly adding a pharmaceutically acceptable aqueous solution and stirring well to form drug-containing micelles; and
      (d3) Filtering through a 0.2 µm filter, and the filtered solution is the drug-containing micelle subassembly comprising drug-containing micelles;
   Additionally, the step (B) for preparing the second lipophilic drug-containing micelle subassembly comprises the following steps (a4) ~ (d4):
      (a4) Mixing another lipophilic drug with a second solvent and stirring at 200 ~ 500 rpm at room temperature until the other lipophilic drug is completely dissolved;
      (b4) Adding the second pharmaceutically acceptable surfactant and stirring well at 100 ~ 300 rpm to volatilize the second solvent, wherein the hydrophilic-lipophilic balance value (HLB value) of the second surfactant is greater than 10;
      (c4) Once the second solvent completely volatilized, slowly adding a pharmaceutically acceptable aqueous solution and stirring well to form second lipophilic drug-containing micelles; and
      (d4) Filtering through a 0.2 µm filter, and the filtered solution is the second lipophilic drug-containing micelle subassembly comprising second lipophilic drug-containing micelles.

Wherein, in step (c3), the drug-containing micelle is a microstructure formed by the first surfactant, and resveratrol is encapsulated in said drug-containing micelle. In step (c4), the second lipophilic drug-containing micelle is a microstructure formed by the second surfactant, and the other lipophilic drug is encapsulated in said second lipophilic drug-containing micelle.

Preferably, the lipophilic drug is at least one of curcumin, quercetin, puerarin, and other lipophilic drugs except resveratrol, or combination thereof.

Preferably, in steps (a3) and/or (a4), the boiling point(s) of the first solvent and/or the second solvent are/is lower than that of pure water.

Preferably, in steps (a3) and/or (a4), the first solvent and/or the second solvent are/is (a) hydrophilic solvent(s).

Preferably, the hydrophilic solvent is at least one of methanol, ethanol, acetone, and other hydrophilic solvents, or combination thereof.

Preferably, the first solvent and/or the second solvent in steps (a3) and/or (a4) are/is (a) lipophilic solvent(s).

Preferably, the lipophilic solvent is at least one of ether, benzene, chloroform, ethyl acetate, dichloromethane, hexane, and other lipophilic solvents, or combination thereof.

Preferably, in steps (b3) and/or (b4), the first surfactant and/or the second surfactant are/is (a) non-ionic surfactant(s).

Preferably, the non-ionic surfactant is at least one of polysorbate 80 (tween 80), solutol HS 15 (polyoxyl 15 hydroxystearate, 2-hydroxyethyl 12-hydroxyoctadecanoate), polyoxyethylene castor oils, and other non-ionic surfactants, or combination thereof.

Preferably, the polyoxyethylene castor oils are at least one of cremophor ELP, cremophor RH 40, and other polyoxyethylene castor oils, or combination thereof.

Preferably, in steps (a3) and (b3), the weight ratio of the resveratrol to the first surfactant is 1:4 to 1:500.

Preferably, in steps (a4) and (b4), the weight ratio of the other lipophilic drug to the second surfactant is 1:4 to 1:500.

Preferably, in steps (a3) and (c3), the weight ratio of the resveratrol to the pharmaceutically acceptable aqueous solution is 1:400 to 3:50.

Preferably, in steps (a4) and (c4), the weight ratio of the other lipophilic drug to the pharmaceutically acceptable aqueous solution is 1:400 to 3:50.

Preferably, in steps (c3) and/or (c4), the pharmaceutically acceptable aqueous solution is water for injection, aqueous solution for injection, or normal saline.

Preferably, in steps (c3) and/or (c4), the pharmaceutically acceptable aqueous solution comprises a local anesthetic.

Preferably, the local anesthetic is at least one of amides, para-aminobenzoic acid esters, and amino ethers, or combination thereof.

Preferably, the amides are at least one of dibucaine, lidocaine, mepivacaine HCl, bupivacine HCl, pyrrocaine HCl, prilocaine HCl, digammacaine, and oxethazaine, or combination thereof.

Preferably, the para-aminobenzoic acid esters are at least one of butacaine, dimethocaine, and tutocaine, or combination thereof.

Preferably, the amino ethers are at least one of quinisocaine and pramocaine, or combination thereof.

Preferably, in step (c3) and/or (c4), the pharmaceutically acceptable aqueous solution comprises an antioxidant.

Preferably, the antioxidant is at least one of beta-carotene, lutein, lycopene, bilirubin, vitamin A, vitamin C (ascorbic acid), vitamin E, uric acid, nitric oxide, nitroxide, pyruvate, catalase, superoxide dismutase, glutathione peroxidases, N-acetyl cysteine, and naringenin, or combination thereof.

Preparation of a fifth pharmaceutical composition were as follows:
(a5) Mixing resveratrol and another lipophilic drug with a solvent and stirring at 200 ~ 500 rpm at room temperature until resveratrol is completely dissolved;
(b5) Adding a pharmaceutically acceptable surfactant and stirring well at 100 ~ 300 rpm to volatilize the solvent, wherein the hydrophilic-lipophilic balance value (HLB value) of the surfactant is greater than 10;
(c5) Once the solvent volatilized completely, slowly adding a pharmaceutically acceptable aqueous solution and stirring well to form drug-containing micelles and second lipophilic drug-containing micelles; and
(d5) Filtering through a 0.2 µm filter and storing the filtered solution comprising drug-containing micelles and the second lipophilic drug-containing micelles in dark and refrigeration.

The types and ranges of the solvents, surfactants, pharmaceutically acceptable aqueous solutions, and other lipophilic drugs used in the fifth pharmaceutical composition are the same as those used in the fourth pharmaceutical composition. Additionally, the ranges of relative ratios of the ingredients used in the fifth pharmaceutical composition are also the same as those used in the fourth pharmaceutical composition.

Preferably, the pharmaceutically acceptable aqueous solution comprises a local anesthetic and/or an antioxidant.

Preferably, the types and ranges of the local anesthetic and/or antioxidant used in the fifth pharmaceutical composition are the same as those used in the fourth pharmaceutical composition.

### Experiment 5: Determination of the quality of pharmaceutical compositions

### Experiment 5-1: Composition analysis

Letting the pharmaceutical composition stand for at least 20 minutes. If the composition does not separate into layers, further analyzing it by a particle analyzer.

Determining whether the pharmaceutical composition comprises micelles by a particle size analyzer. If the particle diameter of the pharmaceutical composition, after being analyzed by a particle analyzer, is smaller than 250 nm, the PDI value is less than 0.4, the solution of the pharmaceutical composition is deemed clear and transparent when observed by the naked eye, and the light beam can be observed when the solution of the pharmaceutical composition is shined by a laser, then it indicates that the pharmaceutical composition comprises micelles.

If micelles are present in the pharmaceutical composition, the prepared composition is the pharmaceutical composition for reducing localized fat in the present invention
If the pharmaceutical composition does not separate into layers and micelles are present after being let stand, the prepared pharmaceutical composition is the preferable pharmaceutical composition for reducing localized fat in the present invention.

### Experiment 5-2: Determination of the stability of pharmaceutical compositions by analyzing the distribution of particle diameters

Using a particle size analyzer (purchased from Malvem) to determine the distribution of particle diameters and the polydispersity index (PDI). If PDI is less than 0.4, it indicates that the stability of pharmaceutical composition is good, that is, the micelles in the pharmaceutical composition can exist stably.

### Experiment 5-3: Determination of the stability of pharmaceutical compositions by accelerated stability test

The storage condition of the pharmaceutical composition in the present invention is 2~8°C. In order to test the stability of the pharmaceutical compositions, the inventor placed the pharmaceutical compositions in an environment of relatively high temperature and relatively high humidity (temperature 25°C±2°C, relative humidity 60%±5%), observed how long the micelles in the pharmaceutical composition can stably exist in a condition of relatively high temperature, to reckon the shelf life of the pharmaceutical composition at 2~8°C.

If the pharmaceutical composition has a shelf life of n months at a condition of 25°C, then the shelf life of the pharmaceutical composition at a condition of 5°C is 2^{((25-5)/10)} folds of n months. That is, the shelf life of the pharmaceutical composition at a condition of 5°C is 2² folds of n months, that is, 4 folds.

For example, if the shelf life of the pharmaceutical composition is 6 months at a condition of 25°C, then the shelf life of the pharmaceutical composition at a condition of 5°C is 24 months (6 months x 4 folds = 24 months.)
Preferably, the pharmaceutical composition maintains at a state without precipitation for at least 24 hours when it is subjected to accelerated stability test at a condition of temperature of 25°C±2°C, relatively humidity of 60%±5%, and in the absence of direct light.

Preferably, the pharmaceutical composition maintains at a state without precipitation for at least 6 months when it is subjected to accelerated stability test at a condition of temperature of 25°C±2°C, relatively humidity of 60%±5%, and in the absence of direct light.

Preferably, the pharmaceutical composition maintains at a state without precipitation for at least 24 months at a condition of temperature of 2~8°C.

### Experiment 6: Maximum drug loading of drug-containing micelles formed by different non-ionic surfactants

Because the maximum drug loading of drug-containing micelles directly affects the injection volume, it has significant affects on drug volume, side effects, and the burden that have to be tolerated by the localized subcutaneous fat layer (such as subcutaneous fat layer of the face) in a single administration. Thus, this experiment investigates the maximum drug loading of drug-containing micelles formed by different non-ionic surfactants to determine which non-ionic surfactant is the best excipient for preparing the pharmaceutical compositions in the present invention.

Four non-ionic surfactants were selected for this experiment. The five non-ionic surfactants were Kolliphor ELP (also known as polyoxyl 35 castor oil, polyoxyl 35 hydrogenated castor oil, or Cremophor ELP, abbreviated as ELP), Kolliphor HS-15 (HS-15), Cremophor RH 40 (abbreviated as RH 40), and polysorbate 80 (also known as Tween 80).

There are 4 groups in the experiment, which are the ELP group, the HS-15 group, the RH40 group, and the Tween 80 group, respectively.

### Experimental steps:

(a') 2.0 g (an example of a first weight) of resveratrol was mixed with 300~500 mL of dichloromethane, and stirred at 150~500 rpm at room temperature until resveratrol dissolved completely.
(b') 18.0g (an example of a second weight) of one of the non-ionic surfactants mentioned above was added to the solution, and stirred well at 100~300 rpm to volatize dichloromethane; and
(c') A composition with 20 g in total was obtained after the solvent volatized completely; 2 g of the composition was weighed out, and 8 g (an example of a third weight) of normal saline for injection was added and stirred well to obtain a composition to be tested. In the composition to be tested, the concentration of resveratrol was 20 mg/g, and the concentration of the non-ionic surfactant was 18%.

The compositions to be tested from the ELP group, HS-15 group, RH40 group, and Tween 80 group were let stand for at least 20 minutes to observe if stratification occurs. If stratification occurs, it indicates that the concentration of resveratrol is too high, causing the micelles in the stage I composition to burst. That is, the non-ionic surfactant cannot be used to prepare the pharmaceutical compositions in the present invention which comprise as high as 20 mg/g of resveratrol.

Assessment of whether each of the excipients (ELP, HS-15, RH40, and Tween 80) is toxic, and calculate the maximum drug loading of drug-containing micelles formed by each excipient based on the maximum acceptable limits of excipients to be injected set by the pharmacopoeia of different countries.

In order to determine the maximum drug loading of ELP, the inventor further performed Experiment 7 and determined that the maximum drug loading of ELP is greater than or equal to 166.7 mg/g. (As the ratio of resveratrol to ELP is 1:5, the prepared pharmaceutical composition contains 166.7 mg/g of resveratrol.)
The results indicated that ELP is the best excipient to prepare the pharmaceutical compositions in the present invention. The concentration of resveratrol can reach 166.7 mg/g in the pharmaceutical compositions prepared with ELP.

### Experiment 7: Preparation of pharmaceutical compositions with Kolliphor ELP (ELP)

In order to determine both of the appropriate ratio between resveratrol and Kolliphor ELP (ELP), and the maximum drug loading when preparing the pharmaceutical compositions in the present invention with ELP, various ratios between resveratrol and Kolliphor ELP (also known as polyoxyl 35 castor oil, polyoxyl 35 hydrogenated castor oil, or Cremophor ELP, abbreviated as ELP) were used in this experiment to prepare a series of pharmaceutical compositions in the present invention, and the stability analysis thereof were performed.

There are 9 groups in this experiment, that is, the first to the ninth group. The preparation method of pharmaceutical composition in each group was roughly the same as the experimental procedure in Experiment 6. The only differences were the weight of resveratrol (the first weight in step (a')), the weight of ELP (the second weight in step (b')), and the weight of normal saline for injection (the third weight in step (c')). In this experiment, the guideline of adding all of the weight of resveratrol (the first weight), the weight of ELP (the second weight), and the weight of normal saline for injection (the third weight) was shown in Table 1.

In this experiment, the ratios of resveratrol to ELP (weight ratio) in the first group to the ninth group were 1:1, 1:2.5, 1:5, 1:8, 1:10. 1:40, 1:80, 1:200, 1:500, respectively, and the final concentrations of resveratrol in the pharmaceutical compositions prepared in the first to the ninth group were 1000 mg/g, 285.71 mg/g, 166.7 mg/g, 60 mg/g, 30 mg/g, 7.5 mg/g, 3.75 mg/g, 0.5 mg/g, and 0.2 mg/g, respectively. That is, the preparation of pharmaceutical composition in the first to the ninth group, the weight ratios of resveratrol in step (a') to ELP in step (b') (the ratios of the first weight to the second weight) were 1:1, 1:2.5, 1:5, 1:8, 1:10. 1:40, 1:80, 1:200, 1:500, respectively, and after adding the third weight of normal saline for injection in step (c'), the final concentrations of resveratrol in the prepared pharmaceutical compositions were 1000 mg/g, 285.71 mg/g, 166.7 mg/g, 60 mg/g, 30 mg/g, 7.5 mg/g, 3.75 mg/g, 0.5 mg/g, and 0.2 mg/g, respectively. Wherein, when the final concentrations of drug was presented as mg/g, this indicates the number of milligrams of resveratrol per gram of pharmaceutical composition.

Particle size analyzer was utilized to determine if micelles were present in the pharmaceutical compositions, and the particle diameter of the micelles was measured.

To assess the stability of the pharmaceutical compositions, the distribution of particle diameters and the polydispersity index (PDI) were determined by a particle size analyzer. The resveratrol content in the micelles was analyzed by high performance liquid chromatography (HPLC; e.g., HPLC-UV) and defined as the "initial drug content".

The pharmaceutical compositions were subjected to accelerated stability test to observe if stratification occurred when the pharmaceutical compositions were stored at high temperature storage condition (25±2°C) for 3 months. The drug content in the micelles was determined by high performance liquid chromatography (HPLC; e.g., HPLC-UV), and defined as the "drug content after accelerated stability test". The "percentage of drug content" was calculated by dividing the "drug content after accelerated stability test" by the "initial drug content". If the percentage of drug content is greater than or equal to 95%, it indicates the stability of the pharmaceutical composition was excellent.

**Table 1. A sample preparation chart for preparing pharmaceutical compositions with ELP**

| Group | Ratio of resveratrol to ELP (weight ratio) | Final concentration of resveratrol in the pharmaceutical composition (mg/g) |
|---|---|---|
| 1 | 1:1 | 1000 |
| 2 | 1:2.5 | 285.71 |
| 3 | 1:5 | 166.7 |
| 4 | 1:8 | 60 |
| 5 | 1:10 | 30 |
| 6 | 1:40 | 7.5 |
| 7 | 1:80 | 3.75 |
| 8 | 1:200 | 0.5 |
| 9 | 1:500 | 0.2 |

Please refer to Table 2. Table 2 is the stability analysis result of the pharmaceutical compositions. When the weight ratio of resveratrol to ELP is 1:5 to 1:500, as shown in Table 2, micelles are present in different pharmaceutical composition, and the measured particle diameter of the micelles was 10~250 nm. Therefore, the pharmaceutical compositions, in which the weight ratio of resveratrol to ELP is 1:5 to 1:500, are all pharmaceutical compositions for reducing localized fat in the present invention.

In terms of stability, when the ratios of resveratrol to ELP were 1:5 to 1:500, PDI was smaller than 0.4. Thus, in order to prepare the pharmaceutical composition for reducing localized fat in the present invention with better stability, based on 1 weight unit defined as the weight of resveratrol, the weight of ELP should be greater than or equal to 5 weight units. Preferably, based on 1 weight unit defined as the weight of resveratrol, the weight of ELP is 5~500 weight units. Preferably, based on 1 weight unit defined as the weight of resveratrol, the weight of ELP is 10~80 weight units. Preferably, based on 1 weight unit defined as the weight of resveratrol, the weight of ELP is 8~80 weight units.

**Table 2. Stability analysis result of the pharmaceutical compositions**

| Group | Ratio of resveratrol to ELP (weight ratio) | Micelle particle diameter (nm) | PDI | Appearance after accelerated stability test | The percentage of drug content (%) after accelerated stability test |
|---|---|---|---|---|---|
| 1 | 1:1 | 404.13 ± 64.70 | 1.00 ± 0.00 | | |
| 2 | 1:2.5 | 787.07 ± 186.84 | 0.71 ± 0.50 | | |
| 3 | 1:5 | 240.63 ± 9.49 | 0.189 ± 0.06 | Clear without stratification | 97.41 ± 0.17 |
| 4 | 1:8 | 174 ± 1.97 | 0.15 ± 0.05 | | |
| 5 | 1:10 | 15.61 ± 0.39 | 0.180 ± 0.01 | Clear without stratification | 98.32 ± 0.23 |
| 6 | 1:40 | 13.66 ± 0.49 | 0.237 ± 0.03 | Clear without stratification | 95.76 ± 0.66 |
| 7 | 1:80 | 12.22 ± 0.13 | 0.181 ± 0.0 | Clear without stratification | 95.54 ± 0.03 |
| 8 | 1:200 | 12.84 ± 0.29 | 0.17 ± 0.02 | | |
| 9 | 1:500 | 12.08 ± 0.96 | 0.15 ± 0.09 | | |

| | | | | | |
|---|---|---|---|---|---|
| In the table above, blank cells indicate that the contents were not analyzed. | | | | | |

Based on the data in Table 2, when the pharmaceutical compositions in the third and the fifth to the seventh group were stored at 25°C for 3 months, the percentage of resveratrol content in every group was greater than 95% and there was no significant decreasing trend comparing to the initial drug content. This result indicates that the pharmaceutical compositions have excellent stability, and based on the rule of thumb of the accelerated stability test, the pharmaceutical compositions can be stored at 2~8°C in refrigeration for at least 24 months.

### Experiment 8: The effect of pharmaceutical compositions prepared with Cremophor ELP on localized fat reduction and weight reduction

### Example 1: Preparation of a resveratrol pharmaceutical composition with Cremophor ELP and resveratrol

(a' ) 0.4 g (an example of the first weight) of resveratrol was mixed with 20 ~ 70 mL of ethanol and stirred at 200 ~ 500 rpm until resveratrol was completely dissolved;
(b') 4 g (an example of the second weight) of Cremophor ELP was added and stirred well at 100 ~ 300 rpm to volatilize ethanol;
(c') Once ethanol volatilized completely, normal saline for injection was slowly added until the weight of normal saline reached 80 g (an example of the third weight), and stirred well to form drug-containing micelles; and
(d') Filtered through a 0.2 µm filter, and the filtered solution was the resveratrol pharmaceutical composition comprising drug-containing micelles. Stored in dark and refrigeration.

In this particular embodiment, the density of normal saline for injection is 1 g/mL. Thus, the volume of 80 g of normal saline for injection is 80 mL. Accordingly, the concentration of resveratrol in the resveratrol pharmaceutical composition is approximately 5 mg/mL (0.4g ÷ 80 mL =
0.005 g/mL = 5 mg/mL.)

In this particular embodiment, the weight ratio of the first weight of resveratrol to the second weight of Cremophor ELP (surfactant) is 0.4:4. That is, the weight ratio is 1:10.

In this particular embodiment, the weight ratio of the first weight of resveratrol to the third weight of normal saline for injection is 0.4 : 80. That is, the weight ratio is 1: 200.

### Example 2: Preparation of a resveratrol complex pharmaceutical composition with Kolliphor ELP, resveratrol, and green tea extract

(a1' ) 0.36 g (an example of the fourth weight) of resveratrol was mixed with 20 ~ 70 mL of ethanol and stirred at 200 ~ 500 rpm at room temperature until resveratrol was completely dissolved;
(b1') 4 g (an example of the fifth weight) of Cremophor ELP was added and stirred well at 100 ~ 300 rpm to volatilize ethanol;
(c1') Once ethanol volatilized completely, the sixth weight of a first normal saline for injection was slowly added and stirred well at 100 ~ 300 rpm to form drug-containing micelles, wherein the first normal saline for injection comprised 0.04 g (an example of the seventh weight) of green tea extract, and the ingredients in the green tea extract included epigallocatechin gallate;
(c11' )The eighth weight of a second normal saline for injection was added such that the total volume of normal saline for injection was 80 mL (i.e., the total volume of the sixth weight of the first normal saline for injection and the eighth weight of the second normal saline for injection was 80 mL), and stirred well to completely dissolve the second normal saline for injection.
(d1') Filtered through a 0.2 µm filter, and the filtered solution comprising drug-containing micelles was the resveratrol complex pharmaceutical composition. Stored in dark and refrigeration.

In this particular embodiment, the concentration of resveratrol -green tea extract in the resveratrol complex pharmaceutical composition is 5 mg/mL [(0.36g + 0.04g) ÷ 80mL = 0.4g ÷ 80 mL = 0.005 g/mL = 5 mg/mL].

In this particular embodiment, based on the total weight of the green tea extract (0.04 g), the content of epigallocatechin gallate in the green tea extract is 95%.

In this particular embodiment, the weight ratio of the fourth weight of resveratrol to the seventh weight of green tea extract is 0.36 : 0.04. That is, the weight ratio is 9:1.

In this particular embodiment, based on a weight unit defined as the total weight of the fourth weigh of resveratrol and the seventh weight of green tea extract (0.36g + 0.04g = 0.4g), the weight of the fifth weight of surfactant is 10 weight units (4g ÷0.4g = 10).

In this particular embodiment, the density of normal saline for injection is 1 g/mL. Thus, using this particular embodiment, wherein the total volume of the sixth weight of the first normal saline for injection and the eighth weight of the second normal saline for injection is 80 mL, as an example, the total weight of normal saline for injection is 80 g.

Accordingly, based on a weight unit defined as the total weight of the fourth weight of resveratrol and the seventh weight of green tea extract (0.36g + 0.04g = 0.4g), the total weight (80 g) of the sixth weight of the first normal saline for injection and the eighth weight of the second normal saline for injection is 200 weight units (80g ÷0.4g = 200).

### Example 3: Fat reduction efficacy of the resveratrol pharmaceutical composition

Six-week-old male Sprague-Dawley rats were used for the experiment. First, 15 rats were fed with high-fat diet to induce accumulation of subcutaneous fat. Feeding was continued until each rat weighed 330 ± 10g. The rats were randomly assigned into three groups: a high-fat diet control group, a low dosage resveratrol group, and a high dosage resveratrol group, with 5 rats in each group such that there was no statistical difference in the body weight between groups.

The 5 mg/mL resveratrol pharmaceutical composition prepared in Example 1 was injected into the lower inguinal subcutaneous fat layer of rats in the low dosage resveratrol group. Each injected dosage was 10 mg of resveratrol per kilogram of body weight (10 mg/kg); that is, 2 mL of the 5 mg/mL resveratrol pharmaceutical composition prepared in Example 1 was injected per kilogram body weight. The 5 mg/mL resveratrol pharmaceutical composition prepared in Example 1 was injected into the lower inguinal subcutaneous fat layer of rats in the high dosage resveratrol group. Each injected dosage was 20 mg of resveratrol per kilogram of body weight (20 mg/kg); that is, 4 mL of the 5 mg/mL resveratrol pharmaceutical composition prepared in Example 1 was injected per kilogram body weight. Rats in the high-fat diet control group were injected with the same volume of normal saline for injection in the same manner mentioned above.

The aforementioned injection sites were the lower inguinal fat pads of rats. Bilateral injections were performed evenly at the injection sites once a day on day 1, 2, 3, and 4 of the experiment. The rats were fed with high-fat diet during the entire duration of the experiment. Their weight change was recorded daily, and their water and food consumption was recorded weekly. The rats were fasted on day 14 and euthanized with CO₂ on day 15.

The bilateral inguinal fat pads of rats were dissected and weighed, and the amount of lower inguinal fat of each group was calculated. The data were presented as mean ± SD and analyzed by one-way ANOVA. Statistical analysis results were presented as symbols or letters. Different symbols or letters indicate statistical difference (p<0.05), and identical symbols or letters indicate no statistical difference (p>0.05).

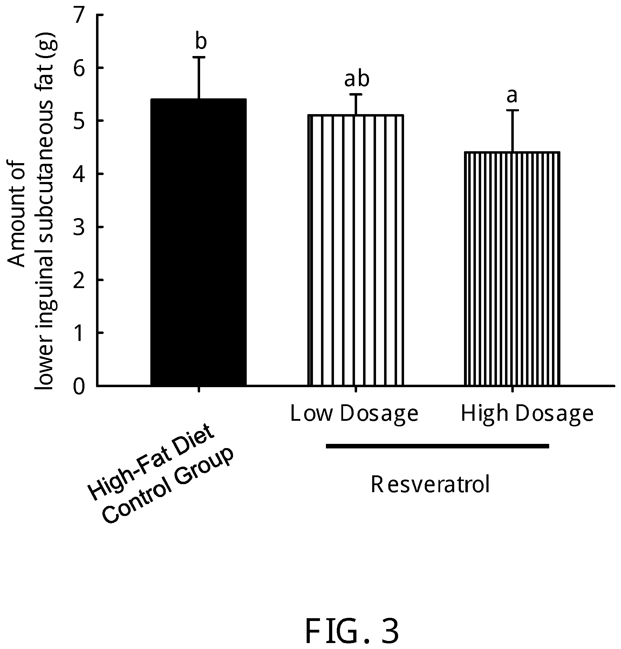

Please refer to Figure 3 and Table 3. Figure 3 is a bar graph showing the effects of the resveratrol pharmaceutical composition on the amount of subcutaneous fat of rats. Table 3 shows the degree of subcutaneous fat reduction in rats caused by the resveratrol pharmaceutical composition. Wherein, the amount of lower inguinal subcutaneous fat is the total weight of bilateral lower inguinal fat pads.

The experimental results demonstrated that comparing to the high-fat diet control group, the amount of lower inguinal subcutaneous fat of rats in the low dosage resveratrol group (wherein the dosage was 10 mg/kg) showed a tendency of reduction, and the localized fat was reduced by 5.6%; the amount of lower subcutaneous inguinal fat of rats in the high dosage resveratrol group (wherein the dosage was 20 mg/kg) were significantly reduced (p<0.05) from that in the high-fat diet control group, and the localized fat was reduced by 18.1%.

**Table 3. The degree of subcutaneous fat reduction in rats caused by the resveratrol pharmaceutical composition**

| Groups | Weight of the lower inguinal subcutaneous fat (g) | Percentage of fat reduction (%) |
|---|---|---|
| | Mean± SD | |
| High-fat diet control group | 5.4± 0.8 | - |
| Low dosage resveratrol group | 5.1± 0.4 | 5.6% |
| High dosage resveratrol group | 4.4± 0.8 | 18.1% |

Based on the experiences of the inventor, if the suitable dosing frequency to be administered to rats is 4 times, the suitable dosing frequency to be administered to human is 1 to 12 times. Preferably, the dosing frequency to be administered to human is 1 to 12 times every other day to every 30 days. Preferably, the dosing frequency to be administered to human is 1 to 6 times every other day to every 30 days.

Based on the experiences of the inventor, if the suitable dosage to be administered to rats is 10 mg/kg ~ 20 mg/kg, the suitable dosage to be administered to human is 0.2 ~ 40 mg/kg.

Preferably, the injected dosage to be administered to human is 0.4~20 mg/kg.

Preferably, the administered dosage to be injected to human is 0.2 ~ 20 mg/cm².

Preferably, the administered dosage to be injected to human is 0.4 ~ 12 mg/cm².

### Example 4: Fat and weight reduction efficacy of the resveratrol complex pharmaceutical composition

Preparation of the green tea extract composition: a 5 mg/mL green tea extract composition was prepared by dissolving green tea extract in normal saline for injection.

Six-week-old male Sprague-Dawley rats were used for the experiment. First, 20 rats were fed with high-fat diet to induce accumulation of subcutaneous fat. Feeding was continued until each rat weighed 330 ± 10g. The rats were randomly assigned into four groups: a high-fat diet control group, a green tea extract group, a resveratrol group, and a resveratrol-green tea extract complex group, with 5 rats in each group such that there was no statistical difference in the body weight between groups. The body weight of each rat was recorded and defined as its "pre-experimental body weight". Then, drugs were administrated as follows:

The 5 mg/mL green tea extract composition prepared in this example was injected into the lower inguinal subcutaneous fat layer of rats in the green tea extract group. Each injected dosage was 10 mg of green tea extract per kilogram of body weight (10 mg/kg); that is, 2 mL of the 5 mg/mL green tea extract composition prepared in this example was injected per kilogram body weight. The 5 mg/mL resveratrol pharmaceutical composition prepared in Example 1 was injected into the lower inguinal subcutaneous fat layer of rats in the resveratrol group. Each injected dosage was 10 mg of resveratrol per kilogram of body weight (10 mg/kg); that is, 2 mL of the 5 mg/mL resveratrol pharmaceutical composition prepared in Example 1 was injected per kilogram body weight. The 5 mg/mL resveratrol complex pharmaceutical composition prepared in Example 2 was injected into the lower inguinal subcutaneous fat layer of rats in the resveratrol-green tea extract complex group. Each injected dosage was 10 mg of resveratrol-green tea extract per kilogram of body weight (10 mg/kg); that is, 2 mL of the 5 mg/mL resveratrol complex pharmaceutical composition prepared in Example 2 was injected per kilogram body weight. Rats in the high-fat diet control group were injected with the same volume of normal saline for injection in the same manner mentioned above.

The aforementioned injection sites were the lower inguinal fat pads of rats. Bilateral injections were performed evenly at the injection sites once a day on day 1, 2, 3, and 4 of the experiment. The rats were fed with high-fat diet during the entire duration of the experiment. Their weight change was recorded daily, and their water and food consumption was recorded weekly. The rats were fasted on day 14 and euthanized with CO₂ on day 15.

The body weight of each rat was recorded and defined as its "post-experimental body weight". The "total body weight gain" of each rat was obtained by subtracting its "pre-experimental body weight" from its "post-experimental body weight". The "relative weight gain" was obtained by dividing the total body weight gain of each group by the total body weight gain of the high fat diet control group.

The bilateral inguinal fat pads of rats were dissected and weighed, and the amount of lower inguinal fat of each group was calculated. The data were presented as mean ± SD and analyzed by one-way ANOVA. Statistical analysis results were presented as symbols or letters. Different symbols or letters indicate statistical difference (p<0.05), and identical symbols or letters indicate no statistical difference (p>0.05).

Because each dosage to be administered to the rats in each group is 10 mg/kg, the efficacy in reducing localized fat shown in the resveratrol-green tea extract complex group should be between the efficacy shown in the resveratrol group and the green tea extract group. If the efficacy in reducing localized fat shown in the resveratrol-green tea extract complex group is better than that in the resveratrol group and the green tea extract group, it indicates that resveratrol and green tea extract in the resveratrol-green tea extract pharmaceutical composition manifest synergy in localized fat reduction efficacy.

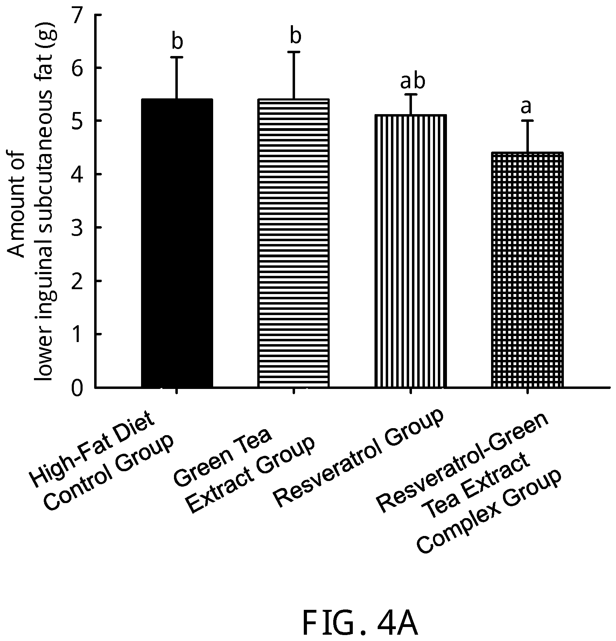

Please refer to Figure 4A and Table 4. Figure 4A is a bar graph showing the effects of the resveratrol complex pharmaceutical composition on the amount of subcutaneous fat of rats. Table 4 shows the degree of subcutaneous fat reduction in rats caused by the resveratrol complex pharmaceutical composition. Wherein, the amount of lower inguinal subcutaneous fat is the total weight of bilateral lower inguinal fat pads.

The experimental results demonstrated in Figure 4A show that comparing to the high-fat diet control group, the subcutaneous fat at the injection site of rats in the green tea extract group (wherein the dosage was 10 mg of green tea extract per kilogram of body weight) was not reduced. Although the amount of subcutaneous fat at the injection site of rats in the resveratrol group did not reach statistical difference comparing to that of the high-fat diet control group, it has shown a tendency of reduction, and the localized fat was reduced by 5.6% (wherein the dosage was 10 mg of resveratrol per kilogram of body weight.) The amount of subcutaneous fat at the injection site of rats in the resveratrol-green tea complex group was reduced significantly (p<0.05), and the localized fat was reduced by 18.9% (wherein the dosage was 10 mg of resveratrol-green tea extract per kilogram of body weight.) That is, the resveratrol complex pharmaceutical composition showed a significantly superior efficacy in reducing localized fat, and the efficacy was 3.4-fold of that of the resveratrol pharmaceutical composition.

This example demonstrated that the resveratrol-green tea extract complex pharmaceutical composition in the present invention has a better localized fat reduction efficacy than the resveratrol simple pharmaceutical composition under the same dosage.

Comparison the localized fat reduction efficacy among the rats in the resveratrol group, the green tea extract group, and the resveratrol-green tea extract complex group, it demonstrated that resveratrol and green tea extract in the resveratrol-green tea extract complex pharmaceutical composition manifests synergy in localized fat reduction efficacy.

**Table 4. The degree of subcutaneous fat reduction in rats caused by the resveratrol complex pharmaceutical composition**

| Groups | Weight of the lower inguinal fat (g) | Percentage of fat reduction (%) |
|---|---|---|
| | Mean± SD | |
| High-fat diet Control group | 5.4± 0.8 | |
| Green tea extract group | 5.4± 0.9 | 0% |
| resveratrol group | 5.1± 0.4 | 5.6% |
| resveratrol-green tea extract complex group | 4.4± 0.6 | 18.9% |

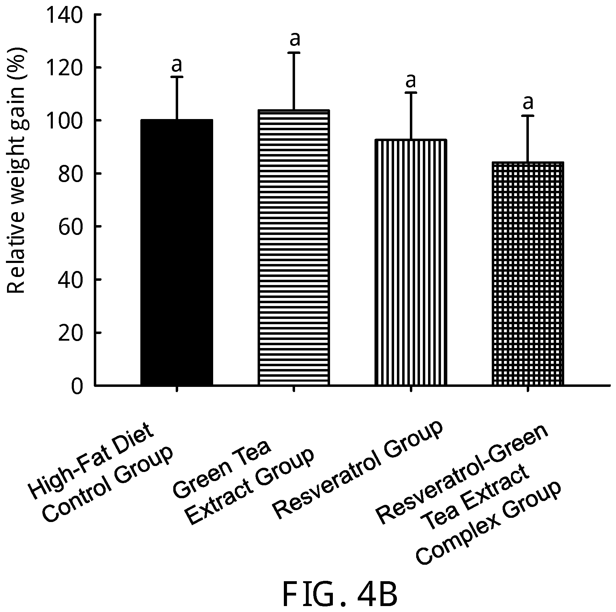

Please refer to Figure 4B and Table 5. Figure 4B is a bar graph showing the effects of the resveratrol complex pharmaceutical composition on the relative weight gain of rats. Table 5 shows the degree of weight reduction in rats caused by the resveratrol complex pharmaceutical composition.

The experimental results demonstrated in Figure 4B show that comparing to the high-fat diet control group, the relative weight gain of rats in the green tea extract group (wherein the dosage was 10 mg of green tea extract per kilogram of body weight) was not reduced. Although the relative weight gain of rats in the resveratrol group did not reach a statistical difference comparing to that of the high-fat diet control group, it has shown a tendency of reduction, and the relative weight gain of rats was reduced by 7.4% (wherein the dosage was 10 mg of resveratrol per kilogram of body weight.) Comparing to the high-fat diet control group, the relative weight gain of rats in the resveratrol-green tea extract complex group was reduced by 15.9% (wherein the dosage was 10 mg of resveratrol-green tea extract complex pharmaceutical composition per kilogram of body weight;) although it did not reach significant difference, the effect of the resveratrol-green tea extract complex group was better than that of resveratrol group. Based on the experiences of the inventor, the resveratrol simple pharmaceutical composition and the resveratrol-green tea extract complex group prepared according to the present invention can achieve significant weight reduction efficacy by increasing the dosage or the frequency of administration.

**Table 5. The degree of weight reduction in rats caused by the resveratrol complex pharmaceutical composition**

| Groups | Relative weight gain (%) | Percentage of weight reduction (%) |
|---|---|---|
| High-fat diet Control group | 100.0± 16.3 | |
| Green tea extract group | 103.7± 21.8 | 0% |
| resveratrol group | 92.6± 17.8 | 7.4% |
| resveratrol-green tea extract complex group | 84.1± 0.6 | 15.9% |

### Experiment 9: The effects of complex pharmaceutical compositions formed by resveratrol and other lipophilic drug on lipolysis

Resveratrol and other lipophilic drugs except resveratrol were used in this experiment to prepare complex pharmaceutical compositions in order to assess the lipolysis efficacy of various lipophilic complex pharmaceutical compositions on mature adipocytes.

In this experiment, puerarin and quercetin were chosen to prepare various lipophilic complex pharmaceutical compositions.

### Experiment 9-1 Cytotoxicity test

Determine if 50 ppm of resveratrol, puerarin, or quercetin have toxicity to cells other than adipocytes by MTT assay. Only if the drug is deemed non-toxic will lipolysis test be proceeded.

Experimental results showed that 50 ppm of resveratrol, puerarin, and quercetin are not cytotoxic to rat somatic cells other than adipocytes, so this dosage will not affect the general somatic cells.

### Experiment 9-2 Lipolysis efficacy on mature adipocytes

A DMSO control group cell culture medium, a resveratrol cell culture medium, a puerarin cell culture medium, a quercetin cell culture medium, a resveratrol-puerarin complex cell culture medium, and a resveratrol-quercetin complex cell culture medium were prepared as follows:

The DMSO control group cell culture medium: DMSO was mixed with an appropriate amount of sterile water to obtain a 0.5% DMSO solution. The 0.5% DMSO solution was mixed with a cell culture medium (product name: Dulbecco's Modified Eagle Medium, purchased from Gibco) to prepare the DMSO control group cell culture medium, wherein, the volume ratio between the 0.5% DMSO solution and the cell culture medium is 1:1000.

The resveratrol cell culture medium: resveratrol was mixed with an appropriate amount of 0.5% DMSO solution to obtain a resveratrol solution. The resveratrol solution was mixed with a cell culture medium (product name: Dulbecco's Modified Eagle Medium, purchased from Gibco) to prepare the resveratrol cell culture medium containing 50 ppm of resveratrol, wherein, the volume ratio between the resveratrol solution and the cell culture medium is 1:1000.

The puerarin cell culture medium: puerarin (purchased from Sigma-Aldrich) was mixed with an appropriate amount of 0.5% DMSO solution to obtain a puerarin solution. The puerarin solution was mixed with a cell culture medium to prepare the puerarin cell culture medium containing 50 ppm of puerarin, wherein, the volume ratio between the puerarin solution and the cell culture medium is 1:1000.

The quercetin cell culture medium: quercetin (purchased from Sigma-Aldrich) was mixed with an appropriate amount of 0.5% DMSO solution to obtain a quercetin solution. The quercetin solution was mixed with a cell culture medium to prepare the quercetin cell culture medium containing 50 ppm of quercetin, wherein, the volume ratio between the puerarin solution and the cell culture medium is 1:1000.

The resveratrol-puerarin complex cell culture medium: resveratrol and puerarin were mixed with an appropriate amount of 0.5% DMSO solution to obtain a resveratrol-puerarin complex solution. Wherein, the weight ratio between resveratrol and puerarin was 2:3. The resveratrol-puerarin complex solution was mixed with a cell culture medium to prepare the resveratrol-puerarin complex cell culture medium containing 50 ppm of resveratrol-puerarin complex drug, wherein, the concentration of resveratrol was 20 ppm, the concentration of puerarin was 30 ppm, and the volume ratio between the resveratrol-puerarin complex solution and the cell culture medium was 1:1000.

The resveratrol-quercetin complex cell culture medium: resveratrol and quercetin were mixed with an appropriate amount of 0.5% DMSO solution to obtain a resveratrol-quercetin complex solution. Wherein, the weight ratio between resveratrol and quercetin was 2:3. The resveratrol-quercetin complex solution was mixed with a cell culture medium to prepare the resveratrol-quercetin complex cell culture medium containing 50 ppm of resveratrol-quercetin complex drug, wherein, the concentration of resveratrol was 20 ppm, the concentration of quercetin was 30 ppm, and the volume ratio between the resveratrol-quercetin complex solution and the cell culture medium was 1:1000.

### Experimental procedure to determine the lipolysis efficacy on mature adipocytes

The adipocyte precursors 3T3-L1 cells (purchased from the Food Industry Research and Development Institute, Taiwan; abbreviated as BCRC) were seeded in 12-well plates, such that each well contained 1x10⁵ cells. After two days of culture, the cells were cultured for another two days in a differentiation induction media (DMI medium, wherein contains 0.5 µM of IBMX (purchased from Sigma-Aldrich), 0.1 µM of dexamethasone (purchased from Sigma-Aldrich), and 5 µg/ml of insulin (purchased from Humulin R.)) Then, the cells were cultured in a medium containing 5 µg/ml of insulin for six days. Once the cell morphology changed from spindle-shaped to spherical and many lipid droplets were accumulated in the cells, it indicates that the cells have differentiated into mature adipocytes.

The mature adipocytes were divided into 6 groups, which are a DMSO control group, a resveratrol group, a puerarin group, a quercetin group, a resveratrol-puerarin complex group, and a resveratrol-quercetin complex group.

The mature adipocytes in the DMSO control group, the resveratrol group, the puerarin group, the quercetin group, the resveratrol-puerarin complex group, and the resveratrol-quercetin complex group were respectively cultured in the DMSO control group cell culture medium, the resveratrol cell culture medium, the puerarin cell culture medium, the quercetin cell culture medium, the resveratrol-puerarin complex cell culture medium, and the resveratrol-quercetin complex cell culture medium for 24 hours.

Annexin V protein (purchased from eBioscience) and propidium iodide (PI; purchased from eBioscience) were mixed with the cells in each group for a period of time, and then the percentage of cells labeled by annexin V protein and PI in each group was analyzed by flow cytometry to assess the percentage of mature adipocytes undergoing apoptosis. Wherein, when a mature adipocyte is labeled by both annexin V protein and PI, it indicates that the cell is undergoing apoptosis; when more mature adipocytes are undergoing apoptosis, it indicates that the lipolysis efficacy of the administered drug is better, and it also indicates that lipolysis is mediated through apoptosis but not necrosis.

The data were presented as mean ± SD and analyzed by one-way ANOVA. Statistical results were shown as symbols or letters. Different symbols or letters indicates statistical difference (p<0.05), and identical symbols or letters indicates no statistical difference (p>0.05).

Because the total amount of the treated drugs in each group is 50 ppm, the apoptosis efficacy of the resveratrol-puerarin complex group should be between the efficacy of the resveratrol group and the puerarin group. If the apoptosis efficacy of the resveratrol-puerarin complex group is better than that of the resveratrol group and the puerarin group, it indicates that resveratrol and puerarin in the resveratrol-puerarin complex pharmaceutical composition manifests synergy in lipolysis efficacy. Similarly, the apoptosis efficacy of the resveratrol-quercetin complex group should be between the efficacy of the resveratrol group and the quercetin group. If the apoptosis efficacy of the resveratrol-quercetin complex group is better than that of the resveratrol group and the quercetin group, it indicates that resveratrol and quercetin in the resveratrol-quercetin complex pharmaceutical composition manifests synergy in lipolysis efficacy.

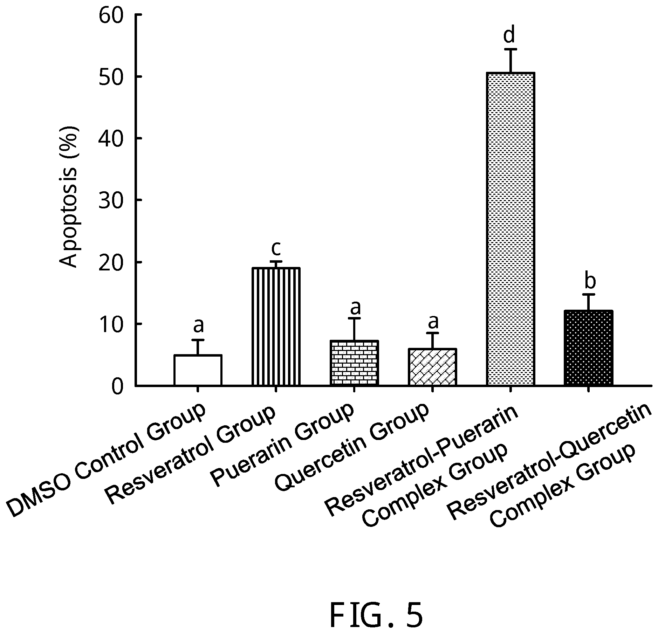

Please refer to Figure 5. Figure 5 is a bar graph showing the effects of the resveratrol-other lipophilic drug complex pharmaceutical composition on the apoptosis of mature adipocytes.

Results in Figure 5 showed that the percentage of apoptotic cells in the DMSO control group was 4.9±2.5%, the percentage of apoptotic cells in the resveratrol group was 19.1±1.1%, the percentage of apoptotic cells in the puerarin group was 7.2±3.7%, the percentage of apoptotic cells in the quercetin group was5.9±2.6%, the percentage of apoptotic cells in the resveratrol-puerarin complex group was 50.6±3.8%, and the percentage of apoptotic cells in the resveratrol-quercetin complex group was 12.1±2.7%.

Comparison the apoptosis efficacy among the resveratrol group, the puerarin group, and the resveratrol-puerarin complex group, it demonstrated that resveratrol and puerarin in the resveratrol-puerarin complex pharmaceutical composition manifests synergy in lipolysis efficacy.

Therefore, complex pharmaceutical compositions formed by resveratrol and various lipophilic drugs can all achieve lipolysis, and there are synergies between resveratrol and various lipophilic drugs in lipolysis efficacy. Thus, the present invention uses resveratrol and various lipophilic drugs to prepare drug-containing micelles and a second lipophilic drug-containing micelles, and further prepares the resveratrol-other lipophilic drug complex pharmaceutical composition, which is a pharmaceutical composition capable to be used for localized lipolysis and weight reduction.

### Experiment 10: The effects of complex pharmaceutical compositions formed by resveratrol and hydrophilic drug on lipolysis

Hydrophilic drug other than green-tea extract and resveratrol were used in this experiment to prepare complex pharmaceutical compositions in order to assess the lipolysis efficacy of various resveratrol-hydrophilic drug complex pharmaceutical compositions on mature adipocytes.

This experiment uses caffeine and L-carnitine to prepare various resveratrol-hydrophilic drug complex pharmaceutical compositions.

### Experiment 10-1 Cytotoxicity test

Determine if 50 ppm of caffeine, and L-carnitine have toxicity to cells other than adipocytes by MTT assay. Only if the drug is deemed non-toxic will lipolysis test be proceeded.

Experimental results showed that 50 ppm of caffeine, and L-carnitine are not cytotoxic to rat somatic cells other than adipocytes, so this dosage will not affect the general somatic cells.

### Experiment 10-2 Lipolysis efficacy on mature adipocytes

A sterile water control group cell culture medium, a resveratrol cell culture medium, a caffeine cell culture medium, a L-carnitine cell culture medium, a resveratrol-caffeine complex cell culture medium, and a resveratrol-L-carnitine complex cell culture medium were prepared as follows:
The sterile water control group cell culture medium: Sterile water was mixed with a cell culture medium to prepare the sterile water control group cell culture medium, wherein, the volume ratio between the sterile water and the cell culture medium is 1:1000.

The resveratrol cell culture medium: it is the same method as the preparation of the resveratrol cell culture medium in Experiment 9-2.

The caffeine cell culture medium: caffeine (purchased from Sigma-Aldrich) was mixed with an appropriate amount of sterile water to obtain a caffeine solution. The caffeine solution was mixed with a cell culture medium to prepare the caffeine cell culture medium containing 50 ppm of caffeine, wherein, the volume ratio between the caffeine solution and the cell culture medium is 1:1000.

The L-carnitine cell culture medium: L-carnitine (purchased from Sigma-Aldrich) was mixed with an appropriate amount of sterile water to obtain a L-carnitine solution. The L-carnitine solution was mixed with a cell culture medium to prepare the L-carnitine cell culture medium containing 50 ppm of L-carnitine, wherein, the volume ratio between the L-carnitine solution and the cell culture medium is 1:1000.

The resveratrol-caffeine complex cell culture medium: resveratrol, caffeine, and an appropriate amount of sterile water were mixed to obtain a resveratrol-caffeine complex solution. Wherein, the weight ratio between resveratrol and caffeine was 2:3. The resveratrol-caffeine complex solution was mixed with a cell culture medium to prepare the resveratrol-caffeine complex cell culture medium containing 50 ppm of resveratrol-caffeine complex drug, wherein, the concentration of resveratrol was 20 ppm, the concentration of caffeine was 30 ppm, and the volume ratio between the resveratrol-caffeine complex solution and the cell culture medium was 1:1000.

The resveratrol-L-carnitine complex cell culture medium: resveratrol, L-carnitine, and an appropriate amount of sterile water were mixed to obtain a resveratrol-L-carnitine complex solution. Wherein, the weight ratio between resveratrol and L-carnitine was 2:3. The resveratrol-L-carnitine complex solution was mixed with a cell culture medium to prepare the resveratrol-L-carnitine complex cell culture medium containing 50 ppm of resveratrol-L-carnitine complex drug, wherein, the concentration of resveratrol was 20 ppm, the concentration of L-carnitine was 30 ppm, and the volume ratio between the resveratrol-L-carnitine complex solution and the cell culture medium was 1:1000.

The preparation of mature adipocytes is the same method as that in Experiment 9-2.

The mature adipocytes were divided into 6 groups, which are a sterile water control group, a resveratrol group, a caffeine group, a L-carnitine group, a resveratrol-caffeine complex group, and a resveratrol-L-carnitine complex group.

The mature adipocytes in the sterile water control group, the resveratrol group, the caffeine group, the L-carnitine group, the resveratrol-caffeine complex group, and the resveratrol-L-carnitine complex group were respectively cultured in the sterile water control group cell culture medium, the resveratrol cell culture medium, the caffeine cell culture medium, the L-carnitine cell culture medium, the resveratrol-caffeine complex cell culture medium, and the resveratrol-L-carnitine complex cell culture medium for 24 hours.

Annexin V protein (purchased from eBioscience) and propidium iodide (PI; purchased from eBioscience) were mixed with the cells in each group for a period of time, and then the percentage of cells labeled by annexin V protein and PI in each group was analyzed by flow cytometry to assess the percentage of mature adipocytes undergoing apoptosis. Wherein, when a mature adipocyte is labeled by both annexin V protein and PI, it indicates that the cell is undergoing apoptosis; when more mature adipocytes are undergoing apoptosis, it indicates that the lipolysis efficacy of the administered drug is better, and it also indicates that lipolysis is mediated through apoptosis but not necrosis.

Because the total amount of the treated drugs in each group is 50 ppm, the ratio of resveratrol thereof was 40%, and the ratio of caffeine thereof was 60%; therefore, the apoptosis efficacy of the resveratrol-caffeine complex group should approximate the average of the efficacy of the resveratrol group and the caffeine group. If the apoptosis efficacy of the resveratrol-caffeine complex group is better than the average of that of the resveratrol group and the caffeine group, it indicates that resveratrol and caffeine in the resveratrol-caffeine complex pharmaceutical composition manifests synergy in lipolysis efficacy. Similarly, because the total amount of the treated drugs in each group is 50 ppm, the ratio of resveratrol thereof was 40%, and the ratio of L-carnitine thereof was 60%; therefore, the apoptosis efficacy of the resveratrol-L-carnitine complex group should approximate the average of the efficacy of the resveratrol group and the L-carnitine group. If the apoptosis efficacy of the resveratrol-L-carnitine complex group is better than the average of that of the resveratrol group and the L-carnitine group, it indicates that resveratrol and L-carnitine in the resveratrol-L-carnitine complex pharmaceutical composition manifests synergy on lipolysis efficacy.

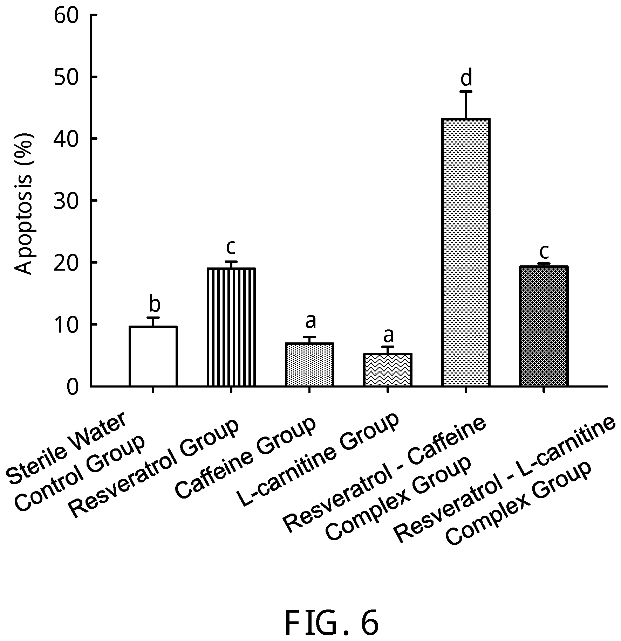

Please refer to Figure 6. Figure 6 shows the effects of resveratrol-other hydrophilic drug complex pharmaceutical compositions on the apoptosis of the mature adipocytes.

Results in Figure 6 showed that the percentage of apoptotic cells in the sterile water control group was 9.6±1.5%, the percentage of apoptotic cells in the resveratrol group was 19.0±1.1%, the percentage of apoptotic cells in the caffeine group was 6.9±1.1%, the percentage of apoptotic cells in the L-carnitine group was 5.2±1.2%, the percentage of apoptotic cells in the resveratrol-caffeine complex group was 43.1±4.5%, and the percentage of apoptotic cells in the resveratrol-L-carnitine complex group was 19.3±0.5%.

Comparison the apoptosis efficacy among the resveratrol group, the caffeine group, and the resveratrol-caffeine complex group, it demonstrated that resveratrol and caffeine in the resveratrol-caffeine complex pharmaceutical composition manifests synergy in lipolysis efficacy.

Comparison between the apoptosis efficacy of the resveratrol group, the L-carnitine group, and the resveratrol-L-carnitine complex group, it demonstrated that resveratrol and L-carnitine in the resveratrol-L-carnitine complex pharmaceutical composition manifests synergy in lipolysis efficacy.

Therefore, complex pharmaceutical compositions formed by resveratrol and various hydrophilic drugs can all achieve lipolysis, and there are synergies between resveratrol and various hydrophilic drugs in lipolysis efficacy. Thus, the present invention uses resveratrol and various hydrophilic drugs to prepare the resveratrol-hydrophilic drug complex pharmaceutical composition comprising drug-containing micelles, which is a pharmaceutical composition capable to be used for localized lipolysis and weight reduction.

As demonstrated by the examples in the present invention, the first pharmaceutical composition, the second pharmaceutical composition, the third pharmaceutical composition, the fourth pharmaceutical composition, the fifth pharmaceutical composition, and other pharmaceutical compositions in the present invention can all reduce the localized fat. Therefore, the first pharmaceutical composition, the second pharmaceutical composition, the third pharmaceutical composition, the fourth pharmaceutical composition, the fifth pharmaceutical composition, and other pharmaceutical compositions in the present invention can be used to prepare subcutaneous implanted devices, subcutaneous implants, solutions for implanted infusion, ointment (or salve), or patches, which is capable to be administered by subcutaneous implantation, implanted infusion, or skin absorption ways, such as applying ointment (or salve) or wearing patches.

Preferably, the first pharmaceutical composition, the second pharmaceutical composition, the third pharmaceutical composition, the fourth pharmaceutical composition, the fifth pharmaceutical composition, and other pharmaceutical compositions in the present invention can reduce the localized fat at the administration site by subcutaneous fat layer injection. Therefore, the first pharmaceutical composition, the second pharmaceutical composition, the third pharmaceutical composition, the fourth pharmaceutical composition, the fifth pharmaceutical composition, and other pharmaceutical compositions in the present invention can be used to prepare formulation for subcutaneous fat layer injection or formulation for subcutaneous injection for reducing localized fat.

## Claims

1. An injection formulation for reducing localized fat or reducing body weight, comprising:
drug-containing micelles;
resveratrol encapsulated in said drug-containing micelles;
wherein the drug-containing micelle is a microstructure formed by a non-ionic surfactant selected from the group consisting of a pharmaceutically acceptable polyoxyethylene castor oil, a polyoxyl 15 hydroxystearate (HS-15), and a combination thereof,
wherein the hydrophilic-lipophilic balance value (HLB value) of the non-ionic surfactant is greater than 10,
wherein the pharmaceutically acceptable polyoxyethylene castor oil comprises polyoxyl 35 castor oil (ELP);
wherein when the non-ionic surfactant comprises the ELP, the weight ratio of the resveratrol to the ELP in the formulation is 1:5 to 1:500;
wherein when the non-ionic surfactant comprises HS-15, the weight ratio of the resveratrol to the HS-15 in the formulation is 1:40; and
wherein the drug-containing micelles do not comprise unsaturated fatty acid.

2. The injection formulation of claim 1, wherein the diameter of the drug-containing micelles is 3~250 nm, optionally wherein the diameter of the drug-containing micelles is 3 ~ 50 nm.

3. The injection formulation of claim 1, wherein the concentration of the resveratrol in the injection formulation is 0.2~ 166.7 mg/mL, optionally wherein the concentration of resveratrol in the injection formulation is 2.5~ 60 mg/mL.

4. The injection formulation of claim 1, wherein the injection formulation further comprises a pharmaceutically acceptable aqueous solution.

5. The injection formulation of any one of claims 1 to 4, wherein the injection formulation further comprises a second lipophilic drug-containing micelle; wherein the second lipophilic drug-containing micelle is another microstructure formed by a second non-ionic surfactant, and a second lipophilic drug, which is encapsulated in said second lipophilic drug-containing micelle,
optionally wherein
a) the second non-ionic surfactant is selected from the group consisting of polysorbate 80, a polyoxyethylene castor oil, polyoxyl 15 hydroxystearate (HS 15), and other non-ionic surfactant, or a combination thereof; further optionally wherein the polyoxyethylene castor oil is at least one of polyoxyl 35 castor oil (ELP), polyoxyl 40 hydrogenated castor oil (RH 40), and other polyoxyethylene castor oil, or combination thereof, or
b) the second lipophilic drug is at least one of quercetin, synephrine, puerarin, curcuminoid, and other lipophilic drug except resveratrol, or combination thereof, further optionally wherein the weight ratio of resveratrol to the second lipophilic drug is 30:1 to 1:20.

6. The injection formulation of any one of claims 1 to 4, wherein the injection formulation further comprises a hydrophilic drug,
optionally wherein the hydrophilic drug is at least one of green tea extract, epigallocatechin gallate, epicatechin, epicatechin gallate, epigallocatechin, gallocatechin gallate, gallocatechin, catechin gallate, catechin, epigallocatechin gallate (EGCG), caffeine, carnitine, L-camitine, synephrine, chlorogenic acid, and other hydrophilic drug, or combination thereof,
further optionally wherein
a) the weight ratio of resveratrol to the hydrophilic drugs is 20:1 to 1:30,
b) wherein the hydrophilic drug is epigallocatechin gallate, and the concentration of epigallocatechin gallate in the injection formulation is 0.25~ 300 mg/mL, or
c) the hydrophilic drug is green tea extract, and the weight ratio of resveratrol to the green tea extract is 30:1 to 1:30.

7. A pharmaceutical composition for use in a method for reducing localized subcutaneous fat or reducing body weight of a subject, wherein the pharmaceutical composition is set forth in any one of claims 1-6, and wherein the method comprising a step of injecting the pharmaceutical composition to a local site of the subject.

8. The pharmaceutical composition for use in a method for reducing localized subcutaneous fat or reducing body weight of a subject according to claim 7, wherein the pharmaceutical composition further comprises the hydrophilic drug,
a) wherein the hydrophilic drug is epigallocatechin gallate, and the
concentration of epigallocatechin gallate in the pharmaceutical composition is 0.25~ 300 mg/mL, or
b) the hydrophilic drugs is green tea extract, and the weight ratio of resveratrol to the green tea extract is 30:1 to 1:30;preferably wherein the amount of the surfactant is 0.24 ~ 70 parts by weight, based on 1 part by weight of the total amount of resveratrol and green tea extract; or the weight ratio of the total weight of the resveratrol and the green tea extract to the surfactant is 4:1 to 1:70.

9. The pharmaceutical composition for use in a method for reducing localized subcutaneous fat or reducing body weight of a subject according to claim 7, wherein
a) the pharmaceutical composition is to be injected at the local site at a dose of 0.2 ~ 20 mg/cm²,
b) the pharmaceutical composition is to be administrated at the local site at a dose of 0.2 ~ 40 mg/kg, or
c) the dosing frequency of the pharmaceutical composition is 1 to 12 times every other day to every 30 days.

10. The pharmaceutical composition for use in a method for reducing localized subcutaneous fat or reducing body weight of a subject according to any one of claims 7 to 9, wherein
the subject is an animal or a human.

## Patentansprüche

1. Eine Injektionsformulierung zur Verringerung von lokalisiertem Fett oder zur Verringerung des Körpergewichts, umfassend:
wirkstoffhaltige Mizellen;
in den wirkstoffhaltigen Mizellen eingekapseltes Resveratrol;
wobei die wirkstoffhaltige Mizelle eine Mikrostruktur ist, die durch ein nichtionisches Tensid gebildet wird, das aus der Gruppe ausgewählt ist,
bestehend aus einem pharmazeutisch verträglichen Polyoxyethylen-Rizinusöl, einem Polyoxyl-15-Hydroxystearat (HS-15) und einer Kombination davon, wobei der Hydrophil-Lipophil-Balance-Wert (HLB-Wert) des nichtionischen Tensids größer als 10 ist,
wobei das pharmazeutisch verträgliche Polyoxyethylen-Rizinusöl Polyoxyl-35-Rizinusöl (ELP) umfasst;
wobei, wenn das nichtionische Tensid ELP umfasst, das Gewichtsverhältnis von Resveratrol zu ELP in der Formulierung 1:5 bis 1:500 beträgt;
wobei, wenn das nichtionische Tensid HS-15 umfasst, das Gewichtsverhältnis von Resveratrol zu HS-15 in der Formulierung 1:40 beträgt; und
wobei die wirkstoffhaltigen Mizellen keine ungesättigten Fettsäuren enthalten.

2. Injektionsformulierung nach Anspruch 1, wobei der Durchmesser der wirkstoffhaltigen Mizellen 3 bis 250 nm beträgt, gegebenenfalls wobei der Durchmesser der wirkstoffhaltigen Mizellen 3 bis 50 nm beträgt.

3. Injektionsformulierung nach Anspruch 1, wobei die Konzentration von Resveratrol in der Injektionsformulierung 0,2 bis 166,7 mg/ml beträgt, wobei die Konzentration von Resveratrol in der Injektionsformulierung gegebenenfalls 2,5 bis 60 mg/ml beträgt.

4. Injektionsformulierung nach Anspruch 1, wobei die Injektionsformulierung ferner eine pharmazeutisch verträgliche wässrige Lösung umfasst.

5. Injektionsformulierung nach einem der Ansprüche 1 bis 4, wobei die Injektionsformulierung ferner eine zweite lipophile, wirkstoffhaltige Mizelle umfasst; wobei die zweite lipophile, wirkstoffhaltige Mizelle eine weitere Mikrostruktur ist, die durch ein zweites nichtionisches Tensid und einen zweiten lipophilen Wirkstoff gebildet wird, welcher in der zweiten lipophilen, wirkstoffhaltigen Mizelle eingekapselt ist,
wobei gegebenenfalls
a) das zweite nichtionische Tensid aus der Gruppe ausgewählt ist, bestehend aus Polysorbat 80, einem Polyoxyethylen-Rizinusöl, Polyoxyl-15-Hydroxystearat (HS 15) und anderen nichtionischen Tensiden oder einer Kombination davon; wobei ferner optional das Polyoxyethylen-Rizinusöl mindestens eines von Polyoxyl-35-Rizinusöl (ELP), Polyoxyl-40-hydriertem Rizinusöl (RH 40) und anderen Polyoxyethylen-Rizinusölen oder einer Kombination davon ist, oder
b) der zweite lipophile Wirkstoff mindestens eines der folgenden ist: Quercetin, Synephrin, Puerarin, Curcuminoid und ein anderer lipophiler Wirkstoff mit Ausnahme von Resveratrol oder eine Kombination davon, wobei ferner optional das Gewichtsverhältnis von Resveratrol zu dem zweiten lipophilen Wirkstoff 30:1 bis 1:20 beträgt.

6. Injektionsformulierung nach einem der Ansprüche 1 bis 4, wobei die Injektionsformulierung ferner einen hydrophilen Wirkstoff enthält,
wobei der hydrophile Wirkstoff gegebenenfalls mindestens einen der folgenden Stoffe umfasst: Grüntee-Extrakt, Epigallocatechingallat, Epicatechin, Epicatechingallat, Epigallocatechin, Gallocatechingallat, Gallocatechin, Catechingallat, Catechin, Epigallocatechingallat (EGCG), Koffein, Carnitin, L-Carnitin, Synephrin, Chlorogensäure und einem anderen hydrophilen Wirkstoff oder einer Kombination davon besteht,
wobei ferner optional
a) das Gewichtsverhältnis von Resveratrol zu den hydrophilen Wirkstoffen 20:1 bis 1:30 beträgt,
b) der hydrophile Wirkstoff Epigallocatechingallat ist und die Konzentration von Epigallocatechingallat in der Injektionsformulierung 0,25 bis 300 mg/ml beträgt, oder
c) der hydrophile Wirkstoff Grüntee-Extrakt ist und das Gewichtsverhältnis von Resveratrol zum Grüntee-Extrakt 30:1 bis 1:30 beträgt.

7. Pharmazeutische Zusammensetzung zur Verwendung in einem Verfahren zur Reduzierung von lokalisiertem subkutanem Fett oder zur Verringerung des Körpergewichts eines Probanden, wobei die pharmazeutische Zusammensetzung in einem der Ansprüche 1 bis 6 beschrieben ist und wobei das Verfahren einen Schritt umfasst, bei dem die pharmazeutische Zusammensetzung an eine lokale Stelle des Probanden injiziert wird.

8. Pharmazeutische Zusammensetzung zur Verwendung in einem Verfahren zur Reduzierung von lokalisiertem subkutanem Fett oder zur Verringerung des Körpergewichts eines Probanden nach Anspruch 7, wobei die pharmazeutische Zusammensetzung ferner den hydrophilen Wirkstoff umfasst,
a) wobei der hydrophile Wirkstoff Epigallocatechingallat ist und die Konzentration von Epigallocatechingallat in der pharmazeutischen Zusammensetzung 0,25 bis 300 mg/ml beträgt, oder
b) der hydrophile Wirkstoff Grüntee-Extrakt ist und das Gewichtsverhältnis von Resveratrol zum Grüntee-Extrakt 30:1 bis 1:30 beträgt; vorzugsweise wobei die Menge des Tensids 0,24 bis 70 Gewichtsteile beträgt, bezogen auf 1 Gewichtsteil der Gesamtmenge an Resveratrol und Grüntee-Extrakt; oder das Gewichtsverhältnis des Gesamtgewichts von Resveratrol und Grüntee-Extrakt zum Tensid 4:1 bis 1:70 beträgt.

9. Pharmazeutische Zusammensetzung zur Verwendung in einem Verfahren zur Reduzierung von lokalisiertem subkutanem Fett oder zur Verringerung des Körpergewichts eines Probanden nach Anspruch 7, wobei
a) die pharmazeutische Zusammensetzung an der lokalen Stelle in einer Dosis von 0,2 bis 20 mg/cm² injiziert werden soll,
b) die pharmazeutische Zusammensetzung an der lokalen Stelle in einer Dosis von 0,2 bis 40 mg/kg verabreicht werden soll, oder
c) die Verabreichungshäufigkeit der pharmazeutischen Zusammensetzung 1 bis 12 Mal alle zwei Tage bis alle 30 Tage beträgt.

10. Pharmazeutische Zusammensetzung zur Verwendung in einem Verfahren zur Reduzierung von lokalisiertem subkutanem Fett oder zur Verringerung des Körpergewichts eines Probanden nach einem der Ansprüche 7 bis 9, wobei der Proband ein Tier oder ein Mensch ist.

## Revendications

1. Une formulation injectable destinée à réduire la graisse localisée ou à réduire le poids corporel, comprenant :
des micelles contenant un principe actif ;
du resvératrol encapsulé dans lesdites micelles contenant un principe actif ;
dans laquelle la micelle contenant un principe actif est une microstructure formée par un agent tensioactif non ionique choisi dans le groupe constitué par une huile de ricin polyoxyéthylénée pharmaceutiquement acceptable, un hydroxystéarate de polyoxyl 15 (HS-15), et une combinaison de ceux-ci,
dans laquelle la valeur d'équilibre hydrophile-lipophile (valeur HLB) de l'agent tensioactif non ionique est supérieure à 10,
dans laquelle l'huile de ricin polyoxyéthylénée pharmaceutiquement acceptable comprend de l'huile de ricin polyoxyl 35 (ELP) ;
dans laquelle, lorsque l'agent tensioactif non ionique comprend l'ELP, le rapport pondéral entre le resvératrol et l'ELP dans la formulation est de 1:5 à 1:500 ;
dans laquelle, lorsque l'agent tensioactif non ionique comprend du HS-15, le rapport pondéral entre le resvératrol et le HS-15 dans la formulation est de 1:40 ; et
dans laquelle les micelles contenant un principe actif ne contiennent pas d'acide gras insaturé.

2. La formulation injectable selon la revendication 1, dans laquelle le diamètre des micelles contenant le principe actif est de 3 à 250 nm, éventuellement dans laquelle le diamètre des micelles contenant le principe actif est de 3 à 50 nm.

3. La formulation injectable selon la revendication 1, dans laquelle la concentration en resvératrol dans la formulation injectable est de 0,2 à 166,7 mg/mL, éventuellement dans laquelle la concentration en resvératrol dans la formulation injectable est de 2,5 à 60 mg/mL.

4. La formulation injectable selon la revendication 1, dans laquelle la formulation injectable comprend en outre une solution aqueuse pharmaceutiquement acceptable.

5. La formulation injectable selon l'une quelconque des revendications 1 à 4, dans laquelle la formulation injectable comprend en outre une deuxième micelle contenant un principe actif lipophile ; dans laquelle la deuxième micelle contenant un principe actif lipophile est une autre microstructure formée par un deuxième agent tensioactif non ionique et un deuxième principe actif lipophile, lequel est encapsulé dans ladite deuxième micelle contenant un principe actif lipophile,
éventuellement dans laquelle
a) le deuxième agent tensioactif non ionique est choisi parmi le groupe constitué du polysorbate 80, d'une huile de ricin polyoxyéthylénée, de l'hydroxystéarate de polyoxyl 15 (HS 15) et d'autres agents tensioactifs non ioniques, ou d'une combinaison de ceux-ci ; en outre, éventuellement, l'huile de ricin polyoxyéthylénée est au moins l'une parmi l'huile de ricin polyoxyl 35 (ELP), l'huile de ricin hydrogénée polyoxyl 40 (RH 40) et d'autres huiles de ricin polyoxyéthylénées, ou une combinaison de celles-ci, ou
b) le deuxième principe actif lipophile est au moins l'un parmi la quercétine, la synéphrine, la puérarine, les curcuminoïdes et d'autres principes actifs lipophiles à l'exception du resvératrol, ou une combinaison de ceux-ci, en outre éventuellement, le rapport pondéral entre le resvératrol et le deuxième principe actif lipophile étant de 30:1 à 1:20.

6. La formulation injectable selon l'une quelconque des revendications 1 à 4, dans laquelle la formulation injectable comprend en outre un principe actif hydrophile,
dans laquelle, éventuellement, le principe actif hydrophile est au moins l'un des composés suivants : extrait de thé vert, gallate d'épigallocatéchine, l'épicatéchine, le gallate d'épicatéchine, l'épigallocatéchine, le gallate de gallocatéchine, la gallocatéchine, le gallate de catéchine, la catéchine, le gallate d'épigallocatéchine (EGCG), la caféine, la carnitine, la L-carnitine, la synéphrine, l'acide chlorogénique et d'autres principes actifs hydrophiles, ou une combinaison de ceux-ci,
dans laquelle, en outre, éventuellement :
a) le rapport pondéral entre le resvératrol et les principes actifs hydrophiles est de 20:1 à 1:30,
b) dans laquelle le principe actif hydrophile est l'épigallocatéchine gallate, et la concentration en épigallocatéchine gallate dans la formulation injectable est de 0,25 à 300 mg/mL, ou
c) le principe actif hydrophile est un extrait de thé vert, et le rapport pondéral entre le resvératrol et l'extrait de thé vert est de 30:1 à 1:30.

7. Une composition pharmaceutique destinée à être utilisée dans un procédé visant à réduire la graisse sous-cutanée localisée ou à réduire le poids corporel d'un sujet, dans laquelle la composition pharmaceutique est décrite dans l'une quelconque des revendications 1 à 6, et dans laquelle le procédé comprend une étape consistant à injecter la composition pharmaceutique au niveau d'un site local du sujet.

8. La composition pharmaceutique destinée à être utilisée dans un procédé visant à réduire la graisse sous-cutanée localisée ou à réduire le poids corporel d'un sujet, selon la revendication 7, dans laquelle la composition pharmaceutique comprend en outre le principe actif hydrophile,
a) dans laquelle le principe actif hydrophile est le gallate d'épigallocatéchine, et la concentration en gallate d'épigallocatéchine dans la composition pharmaceutique est de 0,25 à 300 mg/mL, ou
b) le principe actif hydrophile est un extrait de thé vert, et le rapport pondéral entre le resvératrol et l'extrait de thé vert est de 30:1 à 1:30 ; de préférence, la quantité d'agent tensioactif étant de 0,24 à 70 parties en poids, par rapport à 1 partie en poids de la quantité totale de resvératrol et d'extrait de thé vert ; ou le rapport pondéral entre la masse totale du resvératrol et de l'extrait de thé vert et celle de l'agent tensioactif étant de 4:1 à 1:70.

9. La composition pharmaceutique destinée à être utilisée dans un procédé visant à réduire la graisse sous-cutanée localisée ou à réduire le poids corporel d'un sujet, selon la revendication 7, dans laquelle
a) la composition pharmaceutique doit être injectée au niveau du site local à une dose de 0,2 à 20 mg/cm²,
b) la composition pharmaceutique doit être administrée au niveau du site local à une dose de 0,2 à 40 mg/kg, ou
c) la fréquence d'administration de la composition pharmaceutique est de 1 à 12 fois, tous les deux jours à tous les 30 jours.

10. La composition pharmaceutique destinée à être utilisée dans un procédé visant à réduire la graisse sous-cutanée localisée ou à réduire le poids corporel d'un sujet selon l'une quelconque des revendications 7 à 9, dans laquelle
le sujet est un animal ou un être humain.
